# EUROPEAN PATENT APPLICATION

(11) **EP 4 603 599 A1**
(43) Date of publication of application: **20.08.2025**
(21) Application number: 24157362.5
(22) Date of filing: 13.02.2024
(51) Int. Cl.: C12Q 1/6881, G16B 25/10

(54) **IDENTIFICATION OF REACTIVE T CELL RECEPTORS**

(71) Applicant: Deutsches Krebsforschungszentrum Stiftung des öffentlichen Rechts, 69120 Heidelberg (DE); Universität Heidelberg, 69117 Heidelberg (DE)
(72) Inventor: Green, Edward, 69120 Heidelberg (DE); Platten, Michael, 69120 Heidelberg (DE); Tan, Chin Leng, 69120 Heidelberg (DE)
(74) Representative: Altmann Stößel Dick Patentanwälte PartG mbB

(57) **Abstract**

The present invention relates to a method for identifying at least one biomarker of a T-cell reactive to target cells of a subject (reactive T-cells), said target cells presenting at least one T-cell activating antigen, the method comprising (a) determining expression of a plurality of genes in T cells of said subject; (b) determining reactivities of T cell receptors (TCR) of said T cells to said target cells; and, (c) based on the results of steps (a) and (b), identifying at least one biomarker of a reactive T cell. The present invention also relates to a computer-implemented method of training at least one trainable model for identifying a T-cell reactive to target cells of a subject presenting a T-cell activating antigen (reactive T-cell), the method comprising: (A) providing the trainable model; (B) retrieving allocated training data, the allocated training data comprising gene expression data of a plurality of genes of T cells allocated to reactivities of the T cell receptors (TCRs) of said T cells to said target cells; and (C) training the trainable model on the allocated training data. The present invention further relates to further methods, systems, and trained models related thereto.

## Description

The present invention relates to a method for identifying at least one biomarker of a T-cell reactive to target cells of a subject (reactive T-cells), said target cells presenting at least one T-cell activating antigen, the method comprising (a) determining expression of a plurality of genes in T cells of said subject; (b) determining reactivities of T cell receptors (TCR) of said T cells to said target cells; and, (c) based on the results of steps (a) and (b), identifying at least one biomarker of a reactive T cell. The present invention also relates to a computer-implemented method of training at least one trainable model for identifying a T-cell reactive to target cells of a subject presenting a T-cell activating antigen (reactive T-cell), the method comprising: (A) providing the trainable model; (B) retrieving allocated training data, the allocated training data comprising gene expression data of a plurality of genes of T cells allocated to reactivities of the T cell receptors (TCRs) of said T cells to said target cells; and (C) training the trainable model on the allocated training data. The present invention further relates to further methods, systems, and trained models related thereto.

Over recent years, there has been increasing interest in identifying T cells reactive to disease-associated antigens and their T cell receptors (TCRs) for treatment of a variety of diseases, such as cancers. In such a therapy, reactive T cells may be administered as such, or T cells transgenically modified to express e.g. a tumor reactive TCR may be used.

As a source of T cells for identifying e.g. tumor-reactive TCRs, tumor-infiltrating lymphocytes (TILs) have been used. Tumor reactive T cells within a TIL population can in theory be identified by their upregulation of known T cell activation biomarkers such as CD69 and Nur77, although in practice the value of TCRs identified by such an approach has been found to be limited. Further biomarkers of T cell activation have been described, cf. e.g. Cano-Gamez et al., Nat Comm 11:, art. 1801 (2020) (doi.org/10.1038/s41467-020-15543-y), Magen et al., Cell Rep 29(10):3019 (2019), and Oh et al., Cell 181(7):1612 (2020); WO 2022/200456 A1; WO 2022/200457 A1. Moreover, e.g. biomarkers predicting non-response to immune checkpoint blockade (WO2018/209324) and biomarkers for immunotherapy resistance (WO2019/070755) have been described. Recently, activation markers from tumor infiltrating T lymphocytes were described (WO 2021/188954 A1, Lowery et al., Science 375, 877-884 (2022)).

The success of TIL therapy trials in metastatic melanoma shows that TILs contain a fraction of tumor-reactive T cells which can be harnessed for adoptive cell therapy (Rohaan et al., N. Engl. J. Med. 387, 2113-2125 (2022)), although this success is more limited in non-melanoma cancer types 2 where the baseline fraction of experimentally verifiable, tumor-reactive CD8+ T cells is low. While the fraction of tumor-reactive T cells can be enriched prior to reinfusion, this cell expansion process can exhaust the T cells, compromising their tumor killing efficacy (Crompton et al., Immunol. Rev. 257, 264-276 (2014)), and may lead to the loss of clones (Poschke et al., Clin. Cancer Res. 26, 4289-4301 (2020)). In contrast, personalized transgenic T cell therapies seek to identify and reinfuse defined tumor-reactive TCRs, either in patient autologous T cells or iPSC derived, hypoimmunogenic (allogeneic) T cells. While this generates a highly efficacious product, identifying tumor-reactive TCRs is a 'needle in a haystack' problem (Simoni et al., Nature 557, 575-579 (2018)). Biomarkers for identifying reactive T cells, presumed to carry reactive TCRs, were proposed e.g. for pancreatic cancer (Meng et al., Sci. Transl. Med. 15, eadh9562 (2023)), metastatic colorectal cancer (Lowery et al., Science 375, 877-884 (2022)), non-small cell lung cancer (Caushi. et al., Nature 596, 126-132 (2021)) and for gastrointestinal cancer (Zheng et al. Cancer Cell 40, 410-423.e7 (2022)).

There is nonetheless still a need for improved methods for providing T cells reactive to specific antigens, e.g. cancer antigens, and corresponding TCRs. This problem is solved by the embodiments characterized in the claims and described herein below.

In accordance, the present invention relates to a method for identifying at least one biomarker of a T-cell reactive to target cells of a subject (reactive T-cell), said target cells presenting a T-cell activating antigen, the method comprising
(a) determining expression of a plurality of genes in T cells of said subject;
(b) determining reactivities of T cell receptors (TCR) of said T cells to said target cells; and,
(c) based on the results of steps (a) and (b), identifying at least one biomarker of a reactive T cell.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a plurality of cells. The term "plurality" is used herein in its common meaning to relate to a quantity of more than one, i.e. at least two. Thus, a plurality may preferably be a number of two or more, three or more, five or more, ten or more, 25 or more, 50 or more 100 or more, 1000 or more.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods, unless otherwise indicated. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1 % by weight, most preferably less than 0.1% by weight of non-specified component(s).

Basic statistic tools are known in the art. Thus, whether a portion or a feature of a population is statistically significant can be determined without further ado by the person skilled in the art using various well known statistic evaluation tools, e.g., determination of confidence intervals, p-value determination, Student's t-test, Mann-Whitney test etc. Details are found in textbooks, such as Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.05, 0.01, 0.005, or 0.0001.

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, preferably comprising at least one heterologous sequence. The term polynucleotide encompasses single-as well as, partially or completely, double-stranded polynucleotides. Preferably, the polynucleotide is a DNA polynucleotide, which may also be referred to as "DNA"; is an RNA polynucleotide, which may also be referred to as "RNA", or is a molecule comprising deoxyribonucleotides and ribonucleotides, e.g. as a hybrid DNA/RNA duplex, or as a polynucleotide comprising both types of nucleotides covalently connected. Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like. In view of the description herein, template DNA, e.g. for PCR, primers, e.g. sequencing or amplification primers, and probe oligonucleotides are included in the term polynucleotides.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a polynucleotide may e.g. be an ortholog, a paralog, or another homolog of the specific polynucleotide; the polynucleotide variant may also be a mutant of the specific polynucleotide, preferably a naturally occurring mutant, e.g. identified in a cancer cell. Also preferably, said polynucleotide variant is or is derived from a non-naturally occurring allele of the specific polynucleotide. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences. The percent identity values are, preferably, calculated over the entire nucleic acid sequence region of the specifically referenced polynucleotide, preferably as specified herein elsewhere. The polynucleotides of the present invention either consist, essentially consist of, or comprise the aforementioned nucleic acid sequences. Thus, they may contain further nucleic acid sequences as well.

The term "protein" is understood by the skilled person; preferably, the protein comprises at least one amino acid chain, i.e. a polypeptide as specified herein below, more preferably comprises a plurality of polypeptides. Thus, the protein may be a multimer, e.g. a dimer, a trimer, or the like, wherein the polypeptides in the multimer may be connected covalently, e.g. by a disulfide bridge, or non-covalently, e.g. by ionic interactions, hydrophobic interactions, and/or van der Waals interactions. The protein may consist of identical polypeptides, e.g. may be a homodimer, or may comprise at least two non-identical polypeptides, e.g. may be a heterodimer. More preferably, the protein as specified comprises all structural components as indicated comprised in one continuous covalent polypeptide chain, thus, the protein preferably is or is comprised in a fusion polypeptide.

The term "polypeptide", as used herein, refers to a molecule consisting of a plurality of amino acids that are covalently linked to each other by peptide bonds. Polypeptides consisting of less than 20 amino acids covalently linked by peptide bonds may also be referred to as "peptides". Preferably, the polypeptide comprises of from 100 to 1000, more preferably of from 150 to 1000, still more preferably of from 200 to 500, most preferably of from 250 to 400 amino acids. The polypeptide may also be comprised in a fusion polypeptide, i.e. may comprise amino acid sequences in addition to those specifically indicated. Also, the polypeptide may comprise additional, non-peptidic structures, such as at least one glycosylation, lipid conjugation, and the like. Thus, unless specifically indicated otherwise, reference to specific polypeptides herein preferably includes polypeptide variants.

As used herein, the term "polypeptide variant" relates to any chemical molecule comprising at least one polypeptide as specified herein differing in structure from a specifically indicated polypeptide. Preferably, the polypeptide variant comprises a polypeptide having a contiguous amino acid sequence corresponding to at least 70%, preferably at least 80%, more preferably at least 90%, even more preferably at least 95%, still more preferably at least 98%, most preferably at least 99%, of the amino acid sequence of the polypeptide specifically indicated. Moreover, a polypeptide variant as referred to herein may have an amino acid sequence which differs due to at least one amino acid substitution, deletion and/or addition, wherein the amino acid sequence of the variant is still, preferably, at least 70%, more preferably at least 80%, even more preferably at least 90%, even more preferably at least 95%, still more preferably at least 98%, most preferably at least 99%, identical with the amino acid sequence of the specific polypeptide. The degree of identity between two amino acid sequences can be determined by algorithms well known in the art and as described herein elsewhere. Polypeptide variants referred to above may be allelic variants or any other species specific homologs, paralogs, or orthologs. Moreover, the polypeptide variants referred to herein include fragments of the specific polypeptides or the aforementioned types of polypeptide variants, preferably as long as these fragments and/or variants have the activity as specified. Such fragments may be or may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or activity. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an immunoglobulin. Also, an epitope may be a fragment of an antigen. Also, unless specifically indicated otherwise herein, the compounds specified, in particular the polynucleotides and polypeptides, may be comprised in larger structures.

The "activity" of a polynucleotide or polypeptide as specified herein is, preferably, preserved in the polynucleotide or polypeptide variant; as the skilled person will understand in view of the description herein, the activity of a polynucleotide or polypeptide does not necessarily have to reflect its main natural function, but may preferably be a further activity relevant in the context of the claimed invention. E.g., the activity of a gene product may be to be indicative of a reactive T cell.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide as specified herein, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 90%, preferably at least 95%, more preferably at least 98%, most preferably at least 99%. As will be understood, the term essentially identical includes 100% identity. The aforesaid applies to the term "essentially complementary" mutatis mutandis.

The term "biomarker", as used herein, refers to a molecular species which serves as an indicator for a physiological state as referred to herein. Said molecular species can be a chemical compound which is detectable in a sample of a subject. Moreover, the biomarker may also be a molecular species which is derived from said chemical compound. In such a case, the actual metabolite will be chemically modified in the sample or during the determination process and, as a result of said modification, a chemically different molecular species will be the determined molecular species. E.g., in case the biomarker is a polypeptide or protein, the analyte may be a derivative of the polypeptide or protein, may be a fragment of the polypeptide or protein, or may be a complex of the polypeptide, e.g. an immunocomplex of the polypeptide or a protein comprising more than one polypeptide. Also, the biomarker may be a surrogate marker, i.e. a marker different from the actual biomarker, but nonetheless indicative of the presence and optionally the amount of the biomarker. E.g. the amount of an encoding mRNA may be used as a surrogate marker in case the biomarker is a polypeptide produced by a cell. Further, in case the biomarker has an activity, e.g. a catalytic activity and/or an activating activity, e.g. on immune cells or on target cells, the biomarker may also be determined via said activity, e.g. in an enzymatic assay or an activation assay. Moreover, as is understood by the skilled person, a biomarker according to the present invention need not necessarily correspond to one molecular species. Rather, e.g. in case the biomarker is a polypeptide, the biomarker may comprise polypeptide variant molecular species, e.g. translated from splice variants, glycosylation variants, peptidase processing variants, and the like, wherein, preferably, the variants share at least one determinable feature, e.g. an epitope or an activity. Also, as referred to herein, a biomarker preferably does not necessarily have to be identified by its complete structure and/or nucleic acid or amino acid sequence, provided that the biomarker can be unambiguously allocated to the reactivity of a T cell. Thus, it may be sufficient to identify the biomarker as a compound having one or more particular m/z ratio(s) in mass spectrometry, a particular retention time in a given chromatography, reactivity with a given antibody, or the like.

Preferred biomarkers of the present invention are summarized in Table 1 below, more preferred in the order of Table 1. The biomarkers of Table 1 are, in principle, known to the skilled person and can be accessed by the indicated accession numbers. Unless indicated otherwise, all database accession numbers in this description refer to the respective entry current at the filing date of this description. It is understood by the skilled person that the biomarkers are referenced as biomarkers, not as specific polynucleotides or polypeptides. Accordingly, the biomarkers, in particular polypeptides having the specific sequences deposited under the database accession numbers, are preferably to be understood as exemplary sequences representing a biomarker. Encompassed as gene products as specified herein are also polypeptide variants, encoding polynucleotides, in particular mRNA, and any other gene products deemed appropriate by the skilled person in the context of the present description.

The term "identifying" is used herein in its common meaning of obtaining knowledge of the identity of an item and optionally making said identity known. In accordance, the term "identifying at least one biomarker of a reactive T cell", as used herein, is used in a broad sense including any and all means and methods of providing information on a biomarker of a reactive T cell. In accordance, the biomarker does not have to, but may, be provided in physical form. Thus, identifying a biomarker may comprise providing a dataset indicative that a biomarker is indicative of a reactive T cell. Said biomarker may be a standalone biomarker, i.e. detection of the presence, the absence, or a change in concentration of said biomarker in a T cell may as such be indicative that the T cell is reactive. The biomarker may, however, also be a dependent biomarker, i.e. may be indicative of a reactive T cell in combination with at least one further biomarker. Preferably, the aforesaid dataset is or was determined by single-cell determination of gene expression, preferably by single-cell RNA sequencing. Thus, step a) of the method of identifying at least one biomarker may comprise performing single-cell determination of gene expression of T cells in a sample, wherein expression of at least one gene product is determined. Identifying a biomarker may, however, also comprise physically providing said biomarker, e.g. by purifying or partially purifying said biomarker. Methods of determining biomarker expression in a T cell, preferably in a reactive T cell, are known in the art.

The term "cell" is understood by the skilled person to relate to any cell of the eukaryota, prokarya (bacteria), or archea domains. Preferably, the cell is a living cell, i.e. preferably capable of growth and/or proliferation under appropriate conditions. The term "host cell", as referred to herein, relates to any cell capable of expressing, and preferably presenting on its surface, a TCR polypeptide as specified herein, preferably encoded by a polynucleotide and/or vector. Preferably, the cell is a bacterial cell, more preferably a cell of a common laboratory bacterial strain known in the art, most preferably an Escherichia strain, in particular an E. coli strain. Also preferably, the host cell is a eukaryotic cell, preferably a yeast cell, e.g. a cell of a strain of baker's yeast, or is an animal cell. More preferably, the host cell is an insect cell or a mammalian cell, in particular a mouse or rat cell. Most preferably, the host cell is a human cell. Preferably, the host cell is a T cell, more preferably a CD8+ T cell or a CD4+ T cell, more preferably a CD8+ T cell. As the skilled person understands, a CD4 TCR is preferably expressed in a CD4+ T cell, and a CD8 TCR is preferably expressed in a CD8+ T cell.

The term "target cell", as used herein, relates to a cell having the activity of presenting at least one T cell activating epitope on its surface. In accordance, the target cell expresses MHC-I and/or MHC-II, more preferably MHC-I. Thus, the target cell may be a cell of a body of a subject as specified elsewhere herein; i.e. preferably is a mammalian cell. The target cell may in particular be a cancer cell, preferably presenting at least one cancer antigen. For differentiation, a target cell derived from a metastasis of a cancer may herein also be referred to as a "surrogate target cell", wherein said metastasis preferably is a metastasis from an immune-privileged section of the body. Thus, the surrogate target cell preferably is a cell from a brain metastasis. Preferably, said surrogate target cells comprise at least 80%, preferably at least 90%, more preferably at least 95%, still more preferably at least 98%, of the mutations comprised in tumor cells of the same subject, preferably in tumor cells of the primary tumor. Preferably, the aforesaid mutations are single nucleotide variations; also preferably, the aforesaid said mutations cause expression of at least one cancer epitope, preferably a cancer specific epitope.

The term "tester cell", as used herein, relates to any cell, preferably any eukaryotic cell, more preferably a mammalian cell, most preferably a human cell, expressing a TCR, wherein said TCR preferably is a recombinant TCR comprising at least a pair of CDR3s from a reactive T cell. Thus, for clarity, the tester cell may, however does not have to, be a T cell.

The term "T cell" is understood by the skilled person to relate to a lymphocyte expressing at least one type of T cell receptor. Preferably, the T cell is a CD8+ T cell recognizing major histocompatibility (MHC) class I molecules on the surface of target cells, or is a CD4+ T cell recognizing MHC class II molecules on the surface of target cells. Preferably, the T cell is a cytotoxic T cell, more preferably a CD8+ cytotoxic T cell, which may also be referred to as "killer cell". Also preferably, the T cell is a regulatory or helper T cell, more preferably a regulatory T cell. Preferably, the T cell is reactive to a disease epitope, preferably a cancer epitope, i.e. is a cancer-reactive T cell. Thus, preferably, the T cell expresses a TCR recognizing a disease epitope. Preferably, the T cell is a recombinant cell expressing a chimeric epitope receptor (CAR) and/or a recombinant T cell receptor. Methods of producing appropriate recombinant T cells are known in the art. As is known to the skilled person, human MHCs may also be referred to as "human leukocyte antigens" (HLAs). The T cells preferably is a T cell from a subject and may e.g. be a peripheral T cell. More preferably, the T cell is a tumor-infiltrating lymphocyte (TIL). Also preferably, the T cell is a primary tumor infiltrating and/or a metastasis infiltrating T cell.

The term "complementarity determining region", abbreviated as "CDR", is understood by the skilled person. As is known in the art, each TCR alpha, beta, gamma, and delta chain comprises three CDRs, which comprise the amino acids essentially providing the contacts of a TCR to a peptide presented by an MHC molecule as specified elsewhere herein. As is known to the skilled person, in TCRs, epitope binding specificity is essentially determined by the variable domain and specifically by the CDR3 region of the variable region. Thus, to reproduce the binding properties of a given TCR, it may be sufficient to transfer the CDR3s of said TCR to a suitable backbone, e.g. of another TCR. More preferably, in such case, all CDRs 1 to 3, or the variable regions or fragments thereof, are transferred.

The term "T cell receptor", abbreviated as "TCR", as used herein, relates to a polypeptide complex on the surface of T cells mediating recognition of antigenic peptides presented by target cells, preferably in the context of MHC molecules or MHC-related molecules such as MR1 or CD1, more preferably in the context of MHC molecules, still more preferably in the context of MHC class I or MHC class II molecules, most preferably in the context of MHC class I molecules. Typically, the TCR comprises one TCR-alpha chain and one TCR-beta chain, i.e. is an alpha/beta chain heterodimer. The TCR may, however, also comprise a TCR gamma and a TCR delta chain instead of the TCR alpha and beta chains. The TCR alpha and beta or gamma and delta chains mediate antigen recognition and each comprise a transmembrane region, a constant region, a joining region, and a variable region, the variable region of each TCR alpha, beta, gamma, or delta chain comprising three complementarity determining regions (CDRs), referred to as CDR1, CDR2, and CDR3, respectively. In accordance with usual nomenclature, the complex consisting of an alpha and a beta chain or a gamma and a delta chain is referred to as "T cell receptor" or "TCR" herein, the alpha and/or beta chain and the gamma and/or delta chains commonly or singly being referred to a "TCR polypeptide" or "TCR polypeptides", whereas the polypeptide complex comprising a TCR and accessory polypeptides, such as CD3 and CD247, is referred to as "T cell receptor complex", abbreviated as "TCR complex". Preferably the T cell receptor binds to a major histocompatibility complex (MHC) molecule, preferably an MHC class I or class II, more preferably an MHC class I molecule, presenting an epitope contributing to and/or associated with disease, preferably a cancer epitope. Binding of a T cell receptor to an antigen can be determined by methods known to the skilled person, e.g. by a tetramer assay. Preferably, binding of the TCR to an epitope presented on an MHC activates the T cell. Activation biomarkers of various types of T cells are known in the art and include in particular CD107a, CD69, CD137, CD27, TRAP/CD40L, and CD134. The TCR may also be a soluble TCR. The term "soluble TCR" is, in principle, known to the skilled person to relate to a TCR as specified herein above lacking the transmembrane domains. Thus, preferably, the soluble TCR comprises the constant and the variable regions of the TCR polypeptides of a TCR. More preferably, the soluble TCR comprises the variable regions of the TCR polypeptides of a TCR, preferably in the form of a fusion polypeptide.

The term "reactive T cell", as used herein, relates to a T cell recognizing and being activated by cells presenting a T-cell activating antigen; the reactive T-cell preferably is activated or activatable by cells presenting a T-cell activating antigen, preferably is specifically activated by cells presenting a T-cell activating antigen, the terms "specifically activated by cells presenting a T-cell activating antigen" and "specifically reactive to cells presenting a T-cell activating antigen" indicating that the reactive T-cell preferably is activated by cells presenting a T-cell activating antigen, but not by cells not presenting a T-cell activating antigen, in particular of the same tissue. Activation of T-cells can be measured by methods known in the art, e.g. by measuring cytokine secretion, e.g. interferon-gamma secretion, or by a method as specified herein in the Examples. Preferably, the target cells of the reactive T cell are cancer cells, i.e., the T-cell is reactive to cancer cells, i.e. is a "cancer-reactive T-cell". Thus, preferably, the reactive T-cell expresses a TCR recognizing a cancer antigen, preferably a cancer-specific antigen. In accordance with the above, a T-cell reactive to cancer cells is a T-cell expressing a TCR recognizing a cancer antigen, preferably a cancer-specific antigen.

As indicated, the target cells of the reactive T cell preferably are cancer cells. The term "cancer", as used herein, relates to a disease of an animal, including man, characterized by uncontrolled growth by a group of body cells ("cancer cells"). This uncontrolled growth may be accompanied by intrusion into and destruction of surrounding tissue (invasion) and possibly spread of cancer cells to other locations in the body (metastasis). Preferably, also included by the term cancer is a recurrence of cancer after treatment (relapse). Thus, preferably, the cancer is a solid cancer, a metastasis, or a relapse thereof. Cancer may be induced by an infectious agent, preferably a virus, more preferably an oncogenic virus, more preferably Epstein-Barr virus, a hepatitis virus, Human T-lymphotropic virus 1, a papillomavirus, or Human herpesvirus 8. Cancer may, however, also be induced by chemical compounds, e.g. a carcinogen, by physical factors, such as ionizing radiation, or endogenously, e.g. caused by spontaneous mutation. Preferably, the cancer expresses at least one cancer antigen, preferably a cancer specific antigen, and preferably presents at least one cancer epitope.

Preferably, the cancer is selected from the list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is a solid cancer, a metastasis, or a relapse thereof. More preferably, said cancer is melanoma, brain cancer, preferably metastatic brain cancer, pancreatic cancer, lung cancer, rectal cancer, renal cancer, preferably metastatic renal cancer, or is gastrointestinal cancer.

The term "cancer antigen" relates to an antigen, preferably a polypeptide, expressed by a cancer cell. Preferably, the cancer antigen is expressed at an at least 5fold, preferably at least 10fold, more preferably at least 25fold, lower rate in non-cancer cells compared to cancer cells. Preferably, the cancer antigen is not expressed in non-cancer cells of a subject, preferably of the same tissue, more preferably is not expressed in non-cancer cells of a subject; thus, the cancer antigen preferably is a cancer specific antigen. More preferably, the cancer antigen is a neoantigen and/or comprises a neoepitope, expressed by cancer cells. Preferably, one or more peptides of the cancer antigen are presented via MHC molecules, more preferably MHC class-I molecules, on the surface of cancer cells. More preferably, one or more cancer specific peptides of the cancer antigen (cancer epitopes) are presented via MHC molecules, more preferably MHC class-I molecules, on the surface of cancer cells. As specified elsewhere herein, the cancer preferably is a solid cancer, i.e. a tumor-forming cancer; thus, the cancer antigen preferably is a tumor antigen, more preferably a tumor-specific antigen.

The term "epitope" is known to the skilled person to relate to a (sub-)structure of an antigen which is recognizable for an immune system of a subject, wherein the epitope may be recognizable as such, e.g. to an antibody, or may require accessory factors for recognition; e.g. a T cell epitope may be recognizable to the immune system when presented as an MHC-I or MHC-II complex. As referred to herein, the epitope preferably is a peptide comprising a sequence of amino acids, preferably a contiguous sequence of amino acids. Preferably, the epitope has a length of at least three, more preferably at least four, more preferably at least five, most preferably at least six amino acids. Also preferably, the epitope has a length of at most 50, more preferably at most 25, even more preferably at most 20, most preferably at most 15 amino acids. Thus, preferably, the epitope has a length of from 3 to 50, more preferably from 4 to 25, even more preferably of from 5 to 20, most preferably of from 6 to 15 amino acids. The epitope, in particular the cancer epitope, may be an individual epitope, i.e. identified in a sample of the subject to be treated; it may, however, also be a shared disease epitope, i.e. a disease epitope known or assumed to be shared by a plurality of subjects, e.g. by a plurality of cancers. Shared cancer antigens and corresponding epitopes are well-known in the art.

The epitope as referred to herein is recognized by T cells; thus, the epitope is a T cell epitope. The term "T cell epitope", as used herein, relates to a contiguous sequence of amino acids comprised in a polypeptide, which bind to a major histocompatibility complex (MHC) class I or class II molecule to be presented on the surface of a cell (MHC-I) or of a professional antigen presenting cell (MHC-II). The skilled artisan knows how to predict immunogenic peptides presented on MHC-I or MHC-II (Nielsen et al., (2004), Bioinformatics, 20 (9), 1388-1397), Bordner (2010), PLoS ONE 5(12): e14383) and how to evaluate binding of specific peptides (e.g. Bernardeau et al., (2011), J Immunol Methods, 371(1-2):97-105). Preferably, the T cell epitope is an MHC-I epitope. Preferably, the T cell epitope is an epitope derived from a cancer antigen; thus, preferably, the T cell epitope is an epitope essentially not or not occurring on normal cells of a subject, i.e. on non-cancer cells of said subject.

The term "T cell activating epitope", for which also the expression "activating epitope" may be used, is used herein in a broad sense to relate to any structure presented on the surface of a cell of a subject which can activate a T cell expressing an appropriate TCR. Preferably, the epitope is a polypeptide or fragment thereof, a polysaccharide, or a lipid. More preferably, the epitope is an epitope of a polypeptide presented by said cell of said subject in the context of an MHC molecule, preferably as specified herein above. As the skilled person understands, if a reactive T cell is identified by the method as specified herein in a sample, there preferably is a presumption that there are cells in said subject presenting a T cell activating epitope; since this identification not necessarily includes identifying the T cell activating epitope, the reactive T cell identified and/or its TCR may be used further for identifying the T cell activating epitope. Preferably, the T cell activating epitope is a cancer epitope. Thus, the reactive T cell may in particular be a cancer-reactive T cell or an autoimmune-reactive T cell.

The term "presenting an antigen" is understood by the skilled person to relate to making an antigen, in particular a cancer antigen or an epitope thereof, accessible to the immune system of a subject. In accordance, the term "presenting a T cell activating antigen" is understood by the skilled person as well and preferably relates to making accessible to T cells an activating antigen or an epitope thereof; more preferably, said term relates to presenting an epitope on the surface of a cell, more preferably while the epitope is being bound to a MHC-I or MHC-II molecule on the surface of the presenting cell.

The term "subject", as used herein, relates to an animal, preferably a vertebrate, more preferably a mammal, preferably to a livestock, like a cattle, a horse, a pig, a sheep, or a goat, to a companion animal, such as a cat or a dog, or to a laboratory animal, like a rat, mouse, or guinea pig. Preferably, the mammal is a primate, more preferably a monkey, most preferably a human. Preferably, the subject is suffering from cancer, in particular in case of the method of identifying a T cell reactive to cancer cells of a subject. It is, however, also envisaged that the subject is an apparently healthy subject, preferably at least 50 years, more preferably at least 60 years, more preferably at least 70 years, even more preferably at least 80 years of age.

Identifying a biomarker according to the method described herein comprises determining expression of a plurality of genes, wherein expression of a gene preferable is determined by determining at least one gene product of said gene. The term "gene product", as used herein, includes any and all products of a gene if expressed, including fragments and derivatives of said products. Thus, the gene product preferably is an RNA, more preferably an mRNA, or a derivative or fragment thereof; or a polypeptide, or a derivative or fragment thereof. As the skilled person understands, the amount of a polynucleotide gene product is preferably determined by methods involving hybridization of one or more specific oligonucleotide(s), e.g. PCR primers and/or oligonucleotide probes, to polynucleotides obtained from a sample; appropriate methods are known in the art. Also, determination of the amount of a polypeptide may be achieved by any method deemed appropriate by the skilled person, e.g. by methods involving binding of an antibody. Determining the activity of a gene product, as the skilled person understands, depends on the gene whose product is to be determined; as is also understood by the skilled person, typically the activity of a polypeptide gene product, in particular an enzymatic activity, may be determined. Expression of gene may be determined qualitatively, semi-quantitatively, or quantitatively, which terms are in principle known to the skilled person. Qualitative determination may be a binary assessment that the gene is expressed or not expressed by a T cell, e.g. by determining whether the biomarker is expressed above a detection level of an assay. Semiquantitative determination may comprise allocating an expression level to expression categories, such as low, medium, or high expression. The term quantitative determination is understood by the skilled person to include each and every determination providing information on the amount of a biomarker in a cell and all values derived from such an amount by at least one standard mathematical operation, including in particular calculation of a concentration, of a mean, a median, or an average, normalization, and similar calculations.

Preferably, identifying a biomarker comprises comparing biomarker expression determined in a T cell to a reference. The term "reference", as used herein, refers to expression of a biomarker in a reference population of cells, e.g. an amount of biomarker in a population of reference cells. Preferably, a reference is a threshold value (e.g., an amount or ratio of amounts) for a gene product. The reference may, however, also be a value derived from an amount by any mathematical calculation deemed appropriate by the skilled person, in particular normalization. In accordance with the aforementioned method, a reference is, preferably, a reference obtained from a sample of T cells known to be reactive T cells. In such a case, a value for the biomarker gene product found in a sample being essentially identical to said reference is indicative for a reactive T cell. More preferably, the reference is from a sample of T cells known not be reactive. In such a case, a value for the biomarker gene product found in the T cell to be different with respect to the reference is indicative for the T cell being reactive. The same applies mutatis mutandis for a calculated reference, most preferably the average or median, for the relative or absolute value of the biomarker gene product(s) of a population of non-stimulated T cells. As the skilled person understands, only a small percentage of T cells of any given natural population of T cells will be reactive at a time. In accordance, the above description for a population of T cells known not to be activated may be applied mutatis mutandis to a natural population of T cells of which the activation status is unknown; thus the reference may be obtained from a natural sample of T cells of which reactivity status is unknown. In such a case, a value for the biomarker gene product found in the T cell to be different with respect to the reference is indicative for the T cell being reactive. How to calculate a suitable reference value, preferably, the average or median, is well known in the art. The population of non-stimulated T cells referred to before shall comprise a plurality of T cells, preferably at least 10, more preferably at least 100, still more preferably at least 1,000, most preferably at least 10,000, non-stimulated T cells. The value for a biomarker gene product of T cell of interest and the reference values are essentially identical if the corresponding values are essentially identical. Essentially identical means that the difference between two values is, preferably, not statistically significant. Preferably, the reference(s) are stored in a suitable data storage medium such as a database and are, thus, also available for future assessments.

In accordance with the above, the term "determining reactivities of T cell receptors" relates to determining whether a given TCR binds to an antigen, preferably an epitope, if presented by a target cell. Thus, determining reactivities of T cell receptors may in particular comprise determining binding of an isolated TCR, e.g. a soluble TCR construct, to an antigen, in particular an epitope presented in the context of an MHC, e.g. on a target cell or in vitro; said determining may, however, also comprise determining binding of a T cell carrying said TCR to an antigen, in particular an epitope presented in the context of an MHC, e.g. on a target cell or in vitro, and/or determining activation of said T cell by said antigen, in particular said epitope presented in the context of an MHC, e.g. on a target cell or in vitro.

The method for identifying at least one biomarker comprises step (a) determining expression of a plurality of genes in T cells of said subject. The genes for which expression is determined are selected by the skilled person according to parameters deemed appropriate by the skilled person. E.g. genes may be selected whose expression can be determined by a common assay, e.g. on a hybridization array or an antibody array; or genes may be selected which are not known the be biomarkers, in particular are not known to be biomarkers of reactive T cells; or expression of genes may be determined which are known to cause production of secreted gene products which can be determined in a medium surrounding T cells, and the like. Preferably, genes are selected which can be detected by a commercially available kit and/or system, e.g. a commercially available transcriptomics or proteomics kit. In accordance with the description herein above, step (a) preferably comprises single cell mRNA analysis, preferably of TILs from a subject.

The method for identifying at least one biomarker comprises step (b) determining reactivities of T cell receptors (TCR) of said T cells to said target cells. Options for reactivity testing have been described herein above. As described, reactivity may be tested in a cell-based assay or with isolated components, e.g. soluble TCRs and MHC-tetramers loaded with epitope peptides. In accordance, said determining may be performed using clonal T cells or as single cell assays. For a single cell assay, the method preferably comprises the step of determining the amino acid sequence of at least CDR3 of the TCR of a T cell, preferably of the variable region of a T cell, cloning said amino acid sequence into a TCR sequence, and expressing the resulting recombinant TCR in a suitable tester cell, preferably a suitable tester T cell. Preferably, the reactivities of at least 10, more preferably at least 25, even more preferably at least 50, TCRs to said target cells are determined. As the skilled person understands, determining of a TCR (sub-)sequence may be performed as a substep of step (b) of the method, e.g. on clonally obtained T cells. TCR (sub-)sequences may, however, also be obtained as part of step (a), e.g. in case step (a) is performed as a step of determining mRNAs expressed by said T cell, sequence information on the expressed TCR, including its CDR3 or variable region, may be obtained from said mRNAs expressed.

Preferably, step (b) comprises step (b1) contacting tester T cells expressing said TCRs with target cells. As will be understood by the skilled person, for the purposes of testing reactivity of a TCR, it may be sufficient that the tester cell expresses a TCR comprising at least the CDR3s of the TCR to be tested. Thus, tester cells expressing said TCRs preferably are cells expressing a TCR comprising at least the CDR3 sequences of the TCR to be tested. As is also understood by the skilled person, it is preferable that the TCRs to be tested such that each tester cell expresses one TCR, i.e. all TCR molecules expressed by said tester cell comprise the same pair of CDR3 sequences.

Also preferably, step (b) comprises step (b2) determining said reactivities by determining CD107a+ expression and/or IFN-gamma secretion of tester T cells, and/or lysis of target cells, preferably during and/or after said contacting in step (b1). CD107a+ expression, IFN-gamma secretion, and determining lysis of target cells are well-known methods of detecting T cell activation, appropriate assays are known in the art.

Also preferably, step (b) comprises step (b3) assigning numerical reactivity values to the reactivities of the TCRs determined in step (b), preferably based on the determination in step (b2). The term "assigning numerical reactivity values" is understood by the skilled person. The numerical reactivity value may be a value of any parameter indicative of or correlating with T cell reactivity, or a value derived therefrom by standard mathematical and/or evaluation operations, including in particular multiplication, division, reciprocal formation, scaling, normalization, standardization, error correction, background correction, or mean or median calculation. Well known parameters which may be usable include in particular quantitative measures of CD107a+ expression, IFN-gamma secretion, and lysis of target cells. Thus, the numerical value assigned may e.g. be a measured value of CD107a+ expression, an amount of IFN-gamma secretion and/or a number of cells secreting IFN-gamma, and/or a measure of cell lysis of target cells, such as e.g. %lysis values. Said numerical reactivity values preferably are relative values compared to reactivities determined for further TCRs, e.g. compared to reference values obtained from TCR known to be non-reactive.

The method for identifying at least one biomarker comprises step (c) based on the results of steps (a) and (b), identifying at least one biomarker of a reactive T cell. Said step preferably comprises comparing gene expression data obtained in step (a) to T cell reactivities determined in step (b), and identifying as biomarker those genes whose expression is significantly different in reactive T cells compared to non-reactive T cells. Preferably, step (c) comprises allocating gene expression data of step (a) to T cell reactivities of corresponding TCRs determined in step (b). By said allocation, genes whose expression correlates with TCR reactivity can be identified. Methods for such identification may comprise standard methods of statistical analysis and are, in principle, known in the art.

Preferably, step (c) comprises step (c1) providing a trained computer-implemented trainable model for identifying a reactive T cell, preferably using a computer-implemented training method described herein below.

As the skilled person understands in view of the description herein, all gene products of an identified gene preferably are biomarkers of reactive T cells as referred to herein. It may, however, also be that only one or a few products of a gene are biomarkers, e.g. in cases where only a specific splice variant and/or a translation product thereof are indicative of a reactive T cell, while other products of the same gene are not. Also, it may be that only a specifically processed derivative of a polypeptide gene product is a biomarker as referred to herein, while its unprocessed precursor is not. Nonetheless, unless expressly indicated herein, all gene products of a gene identified according to the method described herein, singly or together, are biomarkers as referred to herein. In particular, for the genes indicated in Table 1, all gene products are indicative of a reactive T cell, i.e. are biomarkers. More preferably, for the genes indicated in Table 1, the mRNA gene products are indicative of a reactive T cell, i.e. are biomarkers.

Advantageously, it was found in the work underlying the present invention that biomarkers of reactive T cells can be identified by allocating single-cell expression data of T cells, in particular TILs, with reactivity data of the respective T cells; said reactivity data can be obtained by subcloning at least the TCR CDR3 regions into TCR heterologous backbones and testing reactivity and/or binding specificity. Using these data, e.g. computer-implemented trainable models may be trained. The resulting trained model may be used to identify reactive T cells and if desired their TCRs, e.g. on a per patient basis. This knowledge can be used to provide reactive T cells for administration to a patient.

The definitions made above apply mutatis mutandis to the following. Additional definitions and explanations made further below also apply for all embodiments described in this specification mutatis mutandis.

The present invention also relates to a computer-implemented method of training at least one trainable model for identifying a T-cell reactive to target cells of a subject presenting a T-cell activating antigen (reactive T-cell), the method comprising:
(A) providing the trainable model;
(B) retrieving allocated training data, the allocated training data comprising gene expression data of T cells allocated to reactivities of the T cell receptors (TCRs) of said T cells to said target cells; and
(C) training the trainable model on the allocated training data.

The present invention also relates to a computer-implemented method of providing a trained model for identifying a reactive T cell, said method comprising the steps of the computer-implemented method of training at least one trainable model as specified herein, and further step (D), thereby providing a trained model for identifying a reactive T cell.

The term "training", as used herein, includes each and every process of establishing parameters of at least one machine learning and/or deep learning model, preferably of an algorithm of said machine learning and/or deep learning model, preferably on at least one training data set, which may also be referred to as set of training data. The training preferably comprises at least one optimization and/or tuning process, wherein a suitable, preferably best, parameter combination, e.g. according to at least one optimization procedure, is determined.

The training method preferably further comprises at least one hyperparameter tuning step for tuning hyperparameters of the trainable model, the term "hyperparameter tuning step" preferably relating to the process of adjusting, preferably optimizing, one or more hyperparameters of the trainable model. Thus, trainable models, e.g. trainable models from standard software libraries such as xgboost or scikit-learn, typically require one or more hyperparameters to be predetermined before starting the training process. Preferably, the hyperparameter tuning comprises Bayesian optimization using scikit-optimize with 10 stratified k-fold cross validation. The hyperparameter tuning step may comprise the pre-adjusting of these hyperparameters, e.g. in order to provide a suitable convergence for the training process. As an example, the allocated training data or at least a part thereof may be used for an initial test for training the trainable model, trying different sets of hyperparameters, wherein the performance of the training process is evaluated on the basis of measurable training performance values such as the speed of convergence, and, wherein, the set of hyperparameters providing a superior performance may finally be chosen for the trainable model. The trainable model may then be trained with this specific set of hyperparameters. The hyperparameter tuning step preferably is performed at least once, preferably before performing step (C). It is, however, also possible to implement a plurality of tuning steps, intermitting with one or more partial training steps, e.g. in order to improve or even optimize the tuning of the hyperparameters during the process.

The term "trainable model", as used herein, relates to any at least one mathematical model configured for transforming one or more input values into one or more output values by using one or more parameters which may be adjusted in order to enable the model to be trained. The trainable model preferably is a trainable mathematical model which is trainable on at least one training data set using one or more of machine learning, deep learning, neural networks, or other form of artificial intelligence. The trainable model preferably can be further trained, optimized or updated based on additional training data. Preferably, the trainable model is trained on a training dataset. The trainable model may be trained by using machine learning. The trainable model may be at least partially data-driven by being trained on data from historical training data.

The trainable model preferably comprises at least one trainable model selected from the group consisting of: a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model. Preferably, the trainable model is trainable XGBoost model.

The term "retrieving", as used herein, relates to any process of obtaining data from a data source. The data source may vary in accordance with the specific application. Thus, in the context of the description herein, retrieving the allocated training data may take place by at least one of the following: downloading the allocated training data from at least one data source, such as from at least one data storage device, from a web- or cloud-based data storage device; obtaining the allocated training data via at least one computer network, such as the Internet; obtaining the allocated training data via at least one wire-based and/or wireless interface. Retrieving of training data may fully or partially take place automatically, such as by automatic download of the allocated training data from public databases, and/or may fully or partially take place manually. Semi-automatic retrieving processes are also possible. Thus, as an example, data from at least one public database may be downloaded, e.g. automatically, and the data may be processed in order to generate the labelled training patient data, e.g. by manual allocation of the training data downloaded. Additionally or alternatively, retrieving the allocated training data may also comprise the actual measurement of the data, e.g., by evaluating one or more samples, such as by determining gene expression data by appropriate measurements.

The term "gene expression data", as used herein, relates to all data pertaining to expressed genes in a biological entity, such as a cell or tissue, preferably in a T cell. Typically, gene expression data are indicative of the abundance of at least one gene product of at least one expressed genes, wherein said gene product may in particular be a polynucleotide, such as an mRNA, or a polypeptide, e.g. of a biomarker. As is known to the skilled person, a particular gene may give rise to more than one polynucleotide gene product, e.g. by alternative splicing, mRNA editing, by being alleles present in the cell, and the like; also, a particular gene may give rise to more than one polypeptide gene product, e.g. via alternative splicing, protein editing, glycosylation, proteolytic processing, and the like. Thus, preferably all gene products originating from one gene are assigned to said gene. It may, however, also be envisaged to assign different gene products to subclasses of one gene, in particular in case said different gene products have different functions in the cell. Also, it may be envisaged to determine only a part of the possible gene products of a gene, e.g. only one gene product, such as the most abundant gene product. Methods for determining abundance of one or more gene products are known in the art and are described elsewhere herein. Preferably, said method for determining abundance of one or more gene products is a method allowing for determining a plurality of gene products in one assay. Thus, in case the genes from which the products shall be determined have not been pre-determined, omics methods, such as transcriptomics or proteomics methods may be used; e.g. abundance of RNAs may be determined by one of the next generation and/or deep transcript sequencing methods known in the art. Based on the nucleic acid sequences of the transcripts or the amino acid sequences of polypeptides of the expressed genes, the encoding genes may be identified, e.g., by comparing the nucleic acid sequences to those nucleic acid sequences of known genes contained in databases. The expressed gene can be identified based on a sequence match or essentially match in a sequence comparison. The name or designation of the identified expressed gene preferably is allocated to the gene expression data as referred to herein. The gene expression data may, in principle, be qualitative data indicating whether a gene is expressed or not, e.g. by determining whether a gene product is detectable above the detection limit of the detection method used. More preferably, the gene expression data are quantitative data comprising information on the abundance of the gene product(s) of the expressed gene, i.e. information as to the amount of gene product determined. In case the genes from which the products shall be determined have been pre-determined, the aforesaid methods may be used as well; however, also methods using specific probes, such as RNA array or protein array methods may be used. Description relating to determining expression of a biomarker herein above applies to determining expression of a gene product mutatis mutandis.

The gene expression data are allocated to the reactivities of TCRs of T cells to target cells. As used herein, the term "reactivity of a TCR" relates to a propensity of a TCR to bind a predetermined epitope presented via an MHC molecule. Methods for determining said propensity are known to the skilled person and include, on an exemplary basis, determining binding of a TCR to a cell, e.g. by using a labeled soluble TCR in a FACS assay; determining activation of a T cell carrying said TCR by a target cell presenting a pre-determined epitope, and/or determining lysis of a target cell presenting a pre-determined epitope by a T cell carrying said TCR. In accordance with the above, the T cells used for determining gene expression data may be enriched for T cells carrying a reactive TCR; said enrichment may be natural enrichment, e.g. by using T cells from a sample known or expected to be enriched for reactive T cells, such as a tumor sample, or by using a sample from a vaccinated subject; said enrichment may, however, also be in vitro enrichment, e.g. by MHC-tetramer staining followed by FACS sorting, by MHC tetramer panning, and/or by cultivation of T cells including, preferably repeated, stimulation with one or more target epitope(s).

The term "allocated", as referred to herein, relates to the property of training data having, besides the gene expression data, additional information permitting a classification of the data. Thus, as an example, the allocated training data may contain, preferably for each patient or each data set of a patient, besides the gene expression data, an indication of the known reactivity and/or activation state of the T cell the gene expression data were obtained from, and/or the reactivity of the corresponding TCR. Thus, as an example, for each T cell comprising a given TCR, besides the gene expression data, further information may be comprised, e.g. indicating the reactivity of the TCR, and/or the reactivity and/or activation state of the T cell comprising said TCR.

Consequently, the allocated training data may be obtained by using data from a plurality of T cells. As an example, data from at least 10 TCRs and/or T cells, preferably data from at least 50 TCRs and/or T cells or more, may be used for generating the allocated training data. Preferably, in case the allocated training data comprise allocated data from TIL, the number of T cells from apparently healthy subjects represented is at least 5fold, preferably at least 10fold, more preferably at least 25 fold, even more preferably at least 50fold, most preferably at least 75 fold, the number of TILs represented. For each T cell, on the one hand, gene expression data may be generated. Further, for each T cell, information on TCR reactivity of the respective T cell may be obtained. As outlined above, also indicators other than gene expression data may be used for obtaining the TCR reactivity of the respective T cell, such as cell surface protein expression levels such as those measured using CITEseq to include common markers of immune cell phenotype such as CD4, CD8, commonly known activation markers, such as CD107a, NFAT, and/or CD69, as well as common markers of immune cell exhaustion such as PD1, CTLA4, and optionally secretion of at least one chemokine, migration behavior, and the like. Then, for each T cell, the gene expression data are allocated at least with the respective information on the TCR reactivity of the respective T cell, in order to obtain an allocated training data set for the respective T cell. The aggregate of the allocated training data set of the TCRs and/or T cells may then form or form part of the allocated training data.

The term "trained model", as used herein, relates to a trainable model which has gone through at least one training process as defined above, by applying, at least once, a set of training data to the trainable model. Preferably, one or more parameters of the trainable model have been adapted during training. Preferably, the trained model is a trainable model which was trained on at least one training dataset. The trained model preferably is or comprises a classifier, configured for classifying an object, on the basis of one or more input variables, describing the object. The trained model preferably comprises at least one trained model selected from the group consisting of: a trained XGBoost model, a trained Adaboost model, a trained Decision Tree model, a trained Catboost model, a trained Gaussian Naive Bayes model, a trained K-nearest neighbour model, a trained Logistic regression model, a trained Radial Basis Function (RBF) Support Vector Machine (SVM) model, a trained Random Forest model, and a trained linear SVM model. Preferably, the trained model is a trained XGBoost model.

The trainable model and/or the trained model preferably may be or may be derived from a model taken from a software library, providing a plurality of trainable models. As an example, the Scikit-learn open-source software library may be mentioned for trainable models and/or trained models being programmed in the Python programming language. Additionally or alternatively, also, as an example, reference may be made to the eXtreme Gradient Boosting (XGBoost) open-source software library, preferably providing gradient boosting trainable models, being programmed in one or more of the programming languages C++, Java, Python, R, Julia, Perl and Scala. Other libraries, however, are also feasible, as well as customized trainable models not retrieved from software libraries.

The term "to classify" is known to the skilled person and preferably relates to any process of assigning an object to one or more predetermined or determinable classes. The classifying preferably comprises a prediction or assignment providing an indication to which class out of a plurality of classes an object belongs, e.g. in accordance with one or more predetermined properties of the object.

As outlined above, the trained model is configured for classifying a TCR and, optionally, a T cell carrying said TCR, into a reactivity state. Thus, as discussed above, the trained model preferably assigns the TCR and optionally the T cell to one reactivity state selected from a predetermined group of at least two (e.g. reactive vs. non-reactive), preferably at least three (e.g. low, intermediate, and strong reactivity), more preferably at least four (e.g. quartiles of reactivity), reactivity states. As indicated elsewhere herein, classification may also be in a continuous parameter, such as a probability of reactivity.

As also outlined above, the trainable model preferably may comprise or may consist of a trainable XGBoost model, i.e. a trainable model implementing a gradient boosting method. As also outlined in further detail below, the trainable XGBoost model preferably may be trained using the following hyperparameters in the following ranges: booster='gbtree'; colsample_bytree of from 0.9 to 1.0; gamma of from 0 to 0.1; max_depth of from 1 to 3; min_child_weight of from 9 to 11; and/or subsample of from 0.05 to 0.3; n_estimators of from 1000 to 1500; learning_rate of from 0.005 to 0.1; and/or scale_pos_weight of from 2 to 6. More preferably, the trainable XGBoost model is trained using the following hyperparameters: booster='gbtree'; colsample_bytree=1.0; gamma=0; max_depth=1; min_child_weight=10; subsample=0.1; n_estimators = 11652; learning_rate = 0.01; and/or scale_pos_weight = 4.68.

As will also be outlined in further detail below, this set of hyperparameters is suited to provide training results leading to a trained XGBoost model on the basis of the allocated training data derived from the above-mentioned studies, the trained XGBoost model being capable of classifying the TCR reactivity with high reliability. For further details on these hyperparameters, as an example, reference may be made to the description of gradient boosting models in the XGBoost library, available e.g. under xgboost.readthedocs.io/en/stable/index.html. Alternatively, however, other libraries may be used, with comparable hyperparameters, as the skilled person will now, such as the Scikit-learn library.

The training method preferably may comprise splitting the allocated training data into a training data set and a test data set. Thus, more preferably, the splitting into a training data set and a test data set may a comprise x%-y% splitting of the allocated training data, with x being in the range 60 to 90, preferably in the range 65 to 75, and more preferably x= 70, and with y=100-x.

The training method may further comprise at least one cross-validation with differing splittings. Thus, a first validation may be performed with an x1 %-y1% splitting of the allocated training data, and at least one second validation may be performed with an x2%-y2% splitting of the allocated training data, with x1≠x2, and the results of the validation may be compared. As an example, a degree of discrepancy between the results obtained for the different splittings may be used for qualifying and/or quantifying the training result.

Additionally or alternatively, the training method may further comprise repeatedly performing step (C) with differing splittings. Again, optionally, the training results, e.g. at least one numeric value qualifying and/or quantifying the training results, e.g. qualifying and/or quantifying the accuracy of the training, may be compared.

As outlined above, the training method preferably may comprise splitting the allocated training data into a training data set and a test data set. The training method may, then, further comprises the following step: at least one validation step, the validation step comprising testing the accuracy of the training by using the test data set.

The testing of the accuracy preferably may comprise using at least one model accuracy metrics. Various model accuracy metrics are generally known in the field of machine learning. Preferably, one or more or even all of the following model accuracy metrics may be used: AUC (area under the curve), overall accuracy, balanced accuracy, and/or weighted f1 score. Other options, however, are also feasible.

The training method may further comprise the following step: determining at least one target set of relevant genes, by using at least one feature extraction method.

Again, various feature extraction methods are generally known to the skilled person in the field of machine learning. As an example, the feature extraction method may comprise considering a permutation feature importance. Additionally or alternatively, the importance of the features may be quantified, such as by quantifying the influence a variation of the value of the respective feature has on the accuracy of the model. The feature extraction method may then, as an example, also comprise considering a feature importance hierarchy. Thus, as an example, the feature extraction method may comprise selecting all features having a feature importance of at least one threshold value. Additionally or alternatively, the feature extraction method may comprise selecting a predetermined number of features highest in ranking with respect to their feature importance. Thus, as an example, the feature extraction method may also comprise, additionally or alternatively, selecting a group of genes of the gene expression data of the T cells with highest TCR reactivity in the feature importance hierarchy to be the target set of relevant genes. Again, additionally or alternatively, the feature extraction method may also comprise selecting a predetermined number of genes of the gene expression data with the highest ranking in the feature importance hierarchy to be the target set of relevant genes. Other means and methods of feature extraction, from the training results, may also be used in addition or as an alternative. Preferably, feature extraction is performed using SHapley Additive exPlanations and feature selection based on SHAP value; corresponding methods are known in the art and have been reviewed e.g. in Lundberg & Lee, "A Unified Approach to Interpreting Model Predictions. in Advances in Neural Information Processing Systems" (eds. Guyon, I. et al.) vol. 30 (Curran Associates, Inc., 2017).

The feature extraction method may be performed such that a reasonable number of genes are selected to be the target set of relevant genes. As an example, for practical applications, selecting a predetermined number of from 5 to 500 genes is generally suitable for diagnostic handling, preferably a predetermined number of from 50 to 100 genes.

In a further aspect of the present invention, a computer-implemented classification method of classifying a TCR reactivity is disclosed. The TCR reactivity is selected from a predetermined group of at least two reactivity states, preferably as detailed herein above. The classification method comprises the following method steps, which, as an example, may be performed in the given order. However, a different order is also feasible. Further, it is possible to perform two or more of the method steps simultaneously or in a fashion overlapping in time. Further, it is also possible to perform one, more than one or even all of the method steps repeatedly.

The classification method comprises the following steps:
(I) retrieving at least one trained model;
(II) retrieving gene expression data from T cells of said subject, the gene expression data comprising at least one predetermined target set of relevant genes; and
(III) applying the trained model to the gene expression data of step (II), thereby assigning a TCR to a reactivity class.

The aforesaid classification method may in particular be a computer-implemented method of predicting reactivity of a T cell receptor to target cells of a subject.

The trained model preferably may be trained by using the training method described herein, such as according to any one of the embodiments described above and/or according to any one of the embodiments described in further detail below. Thus, for most of the terms, definitions and options of the classification method, reference may be made to the training method as described above or as described in further detail below.

The group of predetermined reactivity states preferably is the same as discussed above in the context of the training method. The predetermined target set of relevant genes preferably comprises the target set of relevant genes determined during the training of the trained model. Thus, preferably, as discussed above, the trained model may be trained by using the training method of the present invention. As discussed above in the context of the training method, a target set of relevant genes may be determined during the training. This target set of relevant genes, in the classification method according to the present invention, may be used as the predetermined target set of relevant genes.

Step (III) further may comprise a step of optimization of class separation, e.g. by Jenks natural breaks optimization or Jenk-Fisher Break Optimisation. Also, the classification method may further comprise the following step: (IV) outputting the assigned TCR reactivity state obtained in step (III).

The output, as an example, may take place in one or more of the following fashions: by electronic output, by data transfer, by data storage, by displaying, by acoustic output, by haptic output or by a combination of one or more or even all of the before-mentioned options, thus, as an example, the assigned reactivity state may be displayed on a computer display.

Also, the present invention relates to a system comprising a processor, said processor being configured for performing at least steps (B) and (C) of a method of training at least one trainable model and/or at least step (III) of the method of predicting reactivity of a T cell receptor, both methods as described herein above.

The system configured for performing at least steps (B) and (C) of a method of training at least one trainable model may also be referred to as a training system. The training system comprises at least one processor, the processor being configured, e.g. by software programming, for performing the training method described herein, such as according to any one of the embodiments of the training method described above and/or according to any one of the embodiments of the training method described in further detail below.

The system configured for performing at least step (III) of the method of predicting reactivity of a T cell receptor may also be referred to as a classification system. The classification system comprises at least one processor, the processor being configured, e.g. by software programming, for performing the classification method according to the present invention, such as according to any one of the embodiments of the classification method described above and/or according to any one of the embodiments of the classification method described in further detail below.

As the skilled person will understand in view of the description herein, the system may as well be a combined training and classification system.

The term "processor" is understood by the skilled person. The term preferably may refer, without limitation, to an arbitrary logic circuitry configured for performing basic operations of a computer or system, and/or, generally, to a device which is configured for performing calculations or logic operations. In particular, the processing unit may be configured for processing basic instructions that drive the computer or system. As an example, the processor may comprise at least one arithmetic logic unit (ALU), at least one floating-point unit (FPU), such as a math co-processor or a numeric coprocessor, a plurality of registers, preferably registers configured for supplying operands to the ALU and storing results of operations, and a memory, such as an L1 and L2 cache memory. In particular, the processor may be a multicore processor. Preferably, the processing unit is or comprises a central processing unit (CPU) and/or a graphics processing unit (GPU) . Additionally or alternatively, the processor may be or may comprise a microprocessor, thus preferably the processing unit's elements may be contained in one single integrated circuitry (IC) chip. Additionally or alternatively, the processing unit may be or may comprise one or more application-specific integrated circuits (ASICs) and/or one or more field-programmable gate arrays (FPGAs) or the like. The processing unit preferably may be configured, such as by software programming, for performing one or more evaluation operations.

The term "system" is understood by the skilled person. Preferably, the term includes each and every arbitrary set of interacting or interdependent components parts forming a whole. Preferably, the components interact with each other in order to fulfill at least one common function. The components may be handled independently or may be coupled or connectable. Consequently, the term "training system" generally refers to a system, as defined above, configured for performing at least one training method. Similarly, the term "classification system" generally relates to a system, as defined above, configured for performing at least one classification method.

In a further aspect of the present invention, a computer program is proposed, the computer program comprising instructions which, when the program is executed by at least one processor of a computer or a computer network cause the processor to perform the training method according to the present invention, such as according to any one of the embodiments of the training method described above and/or according to any one of the embodiments of the training method described in further detail below.

In a further aspect of the present invention, a computer program is proposed, the computer program comprising instructions which, when the program is executed by at least one processor of a computer or a computer network cause the processor to perform the classification method according to the present invention, such as according to any one of the embodiments of the classification method described above and/or according to any one of the embodiments of the classification method described in further detail below.

In a further aspect of the present invention, a computer-readable storage medium is proposed, preferably a non-transient computer-readable storage medium, the computer readable storage medium comprising instructions which, when the instructions are executed by at least one processor of a computer or a computer network cause the processor to perform the training method according to the present invention, such as such as according to any one of the embodiments of the training method described above and/or according to any one of the embodiments of the training method described in further detail below.

In a further aspect of the present invention, a computer-readable storage medium is proposed, preferably a non-transient computer-readable storage medium, comprising instructions which, when the instructions are executed by at least one processor of a computer or a computer network cause the processor to perform the classification method according to the present invention, such as according to any one of the embodiments of the classification method described above and/or according to any one of the embodiments of the classification method described in further detail below.

As outlined above, the methods as proposed herein are computer-implemented. As used herein, the terms "computer-readable data carrier" and "computer-readable storage medium" preferably may refer to non-transitory data storage means, such as a hardware storage medium having stored thereon computer-executable instructions. The computer-readable data carrier or storage medium preferably may be or may comprise a storage medium such as a random-access memory (RAM) and/or a read-only memory (ROM).

Thus, preferably, the method steps of the methods as indicated above may be performed by using a computer or a computer network, preferably by using a computer program.

Further disclosed and proposed herein is a computer program product having program code means, in order to perform any one of the methods according to the present invention in one or more of the embodiments enclosed herein when the program is executed on a computer or computer network. Preferably, the program code means may be stored on a computer-readable data carrier and/or on a computer-readable storage medium.

The methods and devices as proposed herein provide a large number of advantages over methods and devices of similar kind. Preferably, the above-mentioned technical challenges may be addressed, wherein, preferably, the accuracy of prediction may be increased. Further, as opposed to diagnosis based on single-platform gene expression data, the present invention also allows for combining different platforms from various studies. Thereby, larger and more diverse training data sets can be achieved which generally may lead to more appropriate and more precise machine learning models.

The present invention also relates to a trained model obtained or obtainable by a computer-implemented method of training at least one trainable model for identifying a reactive T-cell described herein above.

The present invention also relates to a method for treating cancer in a patient, said method comprising
(i) identifying or having identified at least one reactive TCR in a sample of said subject with a trained model described herein and/or a model trained as described herein,
(ii) providing or having provided T cells expressing the at least one TCR identified in step (i);
(iii) administering the T cells of step (ii) to the subject, and thereby
(iv) treating said cancer in said subject.

In the method of treatment, steps (i) and (ii) preferably are in in silico and in vitro steps, respectively, while steps (iii) and (iv) preferably are in vivo steps.

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., those described herein above. Preferably, the treatment shall be effective for at least 25%, at least 50% at least 75%, at least 80%, at least 85%, at least 85%, or at least 90% of the subjects of a given cohort or population. Preferably, treating cancer is reducing tumor burden and/or cancer cell load in a subject. As will be understood by the skilled person, methods and effectiveness of treatment of e.g. cancer is dependent on a variety of factors including, e.g. cancer stage and cancer type. Treating may additionally comprise, e.g. may be preceded, accompanied, and/or followed, by surgery, chemotherapy, radiotherapy, targeted therapy, and/or immunotherapy.

The T cells in step (ii) may be provided in any form deemed appropriate by the skilled person. Preferably, said T cells are provided as a pharmaceutically acceptable preparation fit for administration to a subject. Appropriate pharmaceutically acceptable preparations of T cells are known in the art, as are methods for their administration; e.g. a preparation of T cells may in particular be administered as an i.v. infusion. The T cells of step (ii) may be polyclonal, i.e. T cells expressing non-identical TCRs may be comprised in the same preparation, or the T cells may be monoclonal, i.e. all T cells in the preparation may express the same TCR.

The present invention further relates to a use of a trained model described herein above for identifying at least one reactive TCR.

In view of the above, the following embodiments are particularly envisaged:
Embodiment 1: A method for identifying at least one biomarker of a T-cell reactive to target cells of a subject (reactive T-cell), said target cells presenting a T-cell activating antigen, the method comprising
   (a) determining expression of a plurality of genes in T cells of said subject;
   (b) determining reactivities of T cell receptors (TCR) of said T cells to said target cells; and,
   (c) based on the results of steps (a) and (b), identifying at least one biomarker of a reactive T cell.
Embodiment 2: The method of embodiment 1, wherein in step (a) expression of at least 50, preferably at least 250, more preferably at least 1000, genes is determined.
Embodiment 3: The method of embodiment 1 or 2, wherein step (b) comprises determining the reactivities of at least 10, preferably at least 25, more preferably at least 50, TCRs to said target cells.
Embodiment 4: The method of any one of embodiments 1 to 3, wherein step (b) comprises step (b1) contacting tester T cells expressing said TCRs with target cells.
Embodiment 5: The method of any one of embodiments 1 to 4, wherein each of said TCRs is expressed separately.
Embodiment 6: The method of embodiment 4 or 5, wherein each of said tester T cells expresses one TCR.
Embodiment 7: The method of any one of embodiments 1 to 6, wherein step (b) comprises step (b2) determining said reactivities by determining CD107a+ expression and/or IFN-gamma secretion of tester T cells, and/or lysis of target cells, preferably during and/or after said contacting in step (b1).
Embodiment 8: The method of any one of embodiments 1 to 7, wherein step (b) comprises step (b3) assigning numerical reactivity values to the reactivities of the TCRs determined in step (b), preferably based on the determination in step (b2).
Embodiment 9: The method of embodiment 8, wherein said numerical reactivity values are relative values compared to reactivities determined for further TCRs.
Embodiment 10: The method of embodiment 8, wherein said numerical reactivity values are relative values compared to a reference.
Embodiment 11: The method of any one of embodiments 1 to 10, wherein step (c) comprises allocating gene expression data of step (a) to reactivities of corresponding TCRs determined in step (b).
Embodiment 12: The method of any one of embodiments 1 to 11, wherein step (c) comprises step (c1) providing a trained computer-implemented trainable model for identifying a reactive T cell, preferably using a computer-implemented training method according any one of embodiments 25 to 56 and/or by the method of any one of embodiments 57 to 60.
Embodiment 13: The method of any one of embodiments 1 to 12, wherein said target cells are cancer cells.
Embodiment 14: The method of embodiment 13, wherein said cancer cells form at least one tumor.
Embodiment 15: The method of embodiment 13 or 14, wherein said T cells are tumor infiltrating T cells.
Embodiment 16: The method of any one of embodiments 13 to 15, wherein said T cells are primary tumor infiltrating and/or metastasis infiltrating T cells.
Embodiment 17: The method of any one of embodiments 13 to 16, wherein step (b) comprises contacting a cell expressing said TCR with surrogate target cells being cells of a metastasis of said cancer.
Embodiment 18: The method of embodiment 17, wherein said metastasis is a brain metastasis.
Embodiment 19: The method of embodiment 17 or 18, wherein said surrogate target cells comprise at least 80%, preferably at least 90%, more preferably at least 95%, still more preferably at least 98% of the mutations comprised in tumor cells of the same subject.
Embodiment 20: The method of embodiment 19, wherein said mutations are single nucleotide variations and/or wherein said mutations cause expression of at least one cancer epitope, preferably a cancer specific epitope.
Embodiment 21: The method of any one of embodiments 1 to 20, wherein said subject is a mammal, preferably a human.
Embodiment 22: The method of any one of embodiments 1 to 21, wherein said subject suffers from cancer.
Embodiment 23: The method of any one of embodiments 13 to 22, wherein said cancer formed at least one tumor in said subject.
Embodiment 24: The method of any one of embodiments 13 to 23, wherein said cancer formed at least one metastasis, preferably a brain metastasis, in said subject.
Embodiment 25: A computer-implemented method of training at least one trainable model for identifying a T-cell reactive to target cells of a subject presenting a T-cell activating antigen (reactive T-cell), the method comprising:
   (A) providing the trainable model;
   (B) retrieving allocated training data, the allocated training data comprising gene expression data of a plurality of genes of T cells allocated to reactivities of the T cell receptors (TCRs) of said T cells to said target cells; and
   (C) training the trainable model on the allocated training data.
Embodiment 26: A computer-implemented method of providing a trained model for identifying a reactive T cell, said method comprising the steps of the method of embodiment 25, and further step (D), thereby providing a trained model for identifying a reactive T cell.
Embodiment 27: The method of embodiment 25 or 26, wherein the trainable model is selected from a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model.
Embodiment 28: The method of any one of embodiments 25 to 27, wherein the trainable model is a trainable XGBoost model.
Embodiment 29: The method of any one of embodiments 25 to 28, wherein the reactivities of the TCRs are relative reactivity values compared to the overall reactivities of said T cells, and/or are relative reactivities compared a reference.
Embodiment 30: The method of any one of embodiments 25 to 29, wherein the gene expression data of the allocated training data in step (B) are derived from tumor-infiltrating lymphocytes and are, preferably, obtained by single cell expression analysis, preferably by mRNA analysis.
Embodiment 31: The method of any one of embodiments 25 to 30, wherein the training method comprises at least one hyperparameter tuning step for tuning hyperparameters of the trainable model.
Embodiment 32: The method of embodiment 31, wherein said hyperparameter tuning comprises tuning of parameter colsample_bytree, gamma, learning_rate, max_delta_step, max_depth, min_child_weight, n_estimators, alpha, lambda, scale_pos_weight, and/or subsample.
Embodiment 33: The method of embodiment 31 or 32, wherein said hyperparameter tuning comprises Bayesian optimization using scikit-optimize with 10 stratified k-fold cross validation.
Embodiment 34: The method of any one of embodiments 25 to 33, wherein said method comprises a step of feature selection based on Shapley additive explanation (SHAP) values and repetition of hyperparameter tuning using the selected features.
   The method of any one of embodiments 25 to 34, wherein the trainable model comprises a trainable XGBoost model, wherein the trainable XGBoost model is trained using the following hyperparameters in the following ranges: booster='gbtree'; colsample_bytree of from 0.9 to 1.0; gamma of from 0 to 0.1; max_depth of from 1 to 3; min_child_weight of from 9 to 11; and/or subsample of from 0.05 to 0.3; n_estimators of from 1000 to 1500; learning_rate of from 0.005 to 0.1; and/or scale_pos_weight of from 2 to 6.
Embodiment 35: The method of any one of embodiments 25 to 35, wherein the allocated training data are data obtained from a plurality of different subjects.
Embodiment 36: The method of any one of embodiments 25 to 36, wherein the allocated training data additionally contain information on cell surface protein expression levels, preferably CD4, CD8, CD107a, and/or biomarkers of immune cell exhaustion, preferably PD1 and/or CTLA4.
Embodiment 37: The method of any one of embodiments 25 to 37, wherein the training comprises splitting the allocated training data into a training data set and a test data set.
Embodiment 38: The method of any one of embodiments 25 to 38, wherein the method comprises a cross-validation with differing subsets or folds.
Embodiment 39: The method of any one of embodiments 25 to 39, wherein the method further comprises at least one validation step, the validation step comprising testing the accuracy of the training by using the test data set or using an independent data set.
Embodiment 40: The method of any one of embodiments 25 to 40, wherein the method further comprises determining at least one target set of relevant genes, by using at least one feature extraction method.
Embodiment 41: The method according to embodiment 41, wherein the feature extraction method comprises considering a permutation feature importance.
Embodiment 42: The method of embodiment 41 or 42, wherein the feature extraction method comprises considering a feature importance hierarchy.
Embodiment 43: The method of any one of embodiments 41 to 43, wherein the feature extraction method comprises selecting a group of genes of the gene expression data of the TCRs with the highest ranking in the feature importance hierarchy to be the target set of relevant genes.
Embodiment 44: The method of embodiment 44, wherein the feature extraction method comprises selecting a predetermined number of genes of the gene expression data with the highest ranking in the feature importance hierarchy to be the target set of relevant genes.
Embodiment 45: The method of any one of embodiments 1 to 45, wherein said T-cell activating antigen is a cancer antigen, preferably a cancer-specific antigen.
Embodiment 46: The method of any one of embodiments 25 to 46, wherein the allocated training data comprise data obtained from T cells infiltrating a tumor of at least one subject.
Embodiment 47: The method of any one of embodiments 25 to 47, wherein the allocated training comprise data obtained from T cells infiltrating tumors of a plurality of different subjects.
Embodiment 48: The method of any one of embodiments 25 to 48, wherein the allocated training data comprise data obtained from T cells of at least one apparently healthy subject.
Embodiment 49: The method of any one of embodiments 25 to 49, wherein the allocated training data comprise data obtained from T cells of a plurality of apparently healthy subjects.
Embodiment 50: The method of any one of embodiments 25 to 50, wherein in the allocated training data the number of T cells from apparently healthy subjects represented is at least 5fold, preferably at least 10fold, more preferably at least 25 fold, even more preferably at least 50fold, most preferably at least 75 fold, the number of TILs represented.
Embodiment 51: The method of any one of embodiments 25 to 51, wherein the feature extraction method comprises considering a permutation feature importance.
Embodiment 52: The method of any one of embodiments 25 to 52, wherein the feature extraction method comprises considering a feature importance hierarchy.
Embodiment 53: The method of any one of embodiments 25 to 53, wherein the feature extraction method comprises selecting a group of genes of the gene expression data of the TCRs with the highest ranking in the feature importance hierarchy to be the target set of relevant genes.
Embodiment 54: The method of any one of embodiments 25 to 54, wherein the feature extraction method comprises selecting a predetermined number of genes of the gene expression data with the highest ranking in the feature importance hierarchy to be the target set of relevant genes.
Embodiment 55: The method of any one of embodiments 1 to 55, wherein the plurality of genes is at least 20, preferably at least 30, more preferably at least 38, more preferably at least 48, most preferably is at least 100, preferably wherein said genes are selected from the group consisting of CXCL13, AREG, MAGEH1, FOXP3, ANXA1, HMGB2, MIR4435-2HG, TMSB10, MGAT4A, C12orf57, GIMAP7, HSPE1, IL6ST, STMN1, AC243829.4, IL32, LINC02446, IFNG, NKG7, CTSC, TPT1, LYAR, MIF, PALM2-AKAP2, ISCU, CCR8, UGP2, EEF1B2, SETD7, LTB, CRIP1, APOBEC3G, RBPJ, RGS1, MESD, LINC02099, IKZF3, IFT52, AKIRIN2, RIOK3, CLIC3, ANKRD12, CALR, DDX5, CNST, GNPDA1, PKM, SPOCK2, RSRP1, IL7R, KIR2DL4, MALAT1, CTLA4, TOX, RGL4, KLF6, B2M, IFITM2, ABI3, CST7, PTPN11, BTG1, CCNH, ITSN2, RAD21, CCL3, HMOX1, FYB1, DDX21, GALNT2, HSPD1, GZMA, JAK1, CHST12, ADAM19, PRF1, CD63, TM2D3, CASP8, CARS1, ACP5, PTPN7, RHOH, HAPLN3, CSTB, TLE5, ACTG2, CD7, IL2RA, CD99, CARHSP1, TMSB4X, PSMB9, CCR4, AKAP13, RABAC1, HLA-DRA, FTH1, CD27, and PCM1.
Embodiment 56: A computer-implemented method of predicting reactivity of a T cell receptor to target cells of a subject, the method comprising
   (I) retrieving at least one trained model;
   (II) retrieving gene expression data from T cells of said subject, the gene expression data comprising at least one predetermined target set of relevant genes; and
   (III) applying the trained model to the gene expression data of step (II), thereby predicting reactivities of T cell receptors (TCRs) of said T cells to said target cells.
Embodiment 57: The method of embodiment 57, wherein the trained model has a feature specified in any one of embodiments 25 to 56.
Embodiment 58: The method of embodiment 57 or 58, wherein the trained model is the trained model of embodiment 66.
Embodiment 59: The method of any one of embodiments 57 to 59, further comprising step (IV) outputting the predicted reactivities for said TCRs.
Embodiment 60: The method of embodiment 60, wherein said reactivities are probabilities of being reactive.
Embodiment 61: A system comprising a processor, said processor being configured for performing at least steps (B) and (C) of a method according to any one of embodiments 25 to 56 and/or at least step (III) of the method according to any one of embodiments 57 to 61.
Embodiment 62: The system of embodiment 62 comprising, preferably tangibly embedded, an executable code which, when executed, causes the system to perform at least steps (B) and (C) of a method according to any one of embodiments 25 to 56 and/or at least step (III) of the method according to any one of embodiments 57 to 61.
Embodiment 63: The system of embodiment 62 or 63, further comprising a data storage unit comprising the allocated training data.
Embodiment 64: The system of embodiment 63 or 64, further comprising a code storage unit comprising said executable code.
Embodiment 65: A trained model obtained or obtainable by a method according to any one of embodiments 25 to 56, preferably tangibly embedded on a data carrier.
Embodiment 66: A method for treating cancer in a patient, said method comprising
   (a) identifying or having identified at least one reactive TCR in a sample of said subject with a trained model according to embodiment 60 and/or a model trained according to any one of embodiments 25 to 56,
   (b) providing or having provided T cells expressing the at least one TCR identified in step (i);
   (c) administering the T cells of step (ii) to the subject, and thereby
   (d) treating said cancer in said subject.
Embodiment 67: The method of embodiment 67, wherein said sample is a tumor sample or a blood sample.
Embodiment 68: Use of a trained model according to embodiment 66 for identifying at least one reactive TCR.
Embodiment 69: A computer program comprising instructions which, when the program is executed by at least one processor of a computer or a computer network cause the processor to perform the method according to any one of embodiments 25 to 56 and/or at least step (III) of the method according to any one of embodiments 57 to 61.
Embodiment 70: A computer-readable storage medium, specifically a non-transient computer-readable storage medium, comprising instructions which, when the instructions are executed by at least one processor of a computer or a computer network cause the processor to perform the method according to any one of embodiments 25 to 56 and/or at least step (III) of the method according to any one of embodiments 57 to 61.

All references cited in this specification are herewith incorporated by reference with respect to their entire disclosure content and the disclosure content preferably mentioned in this specification.

### Figure Legends

Fig. 1: Top 50 TCR clones of patient BT21 were co-cultured with BT21 patient-derived cell lines and the expression of CD107a was measured by flow cytometry with mock and DMF5 TCR included as negative control. Insert: enlarged representation for TCR IDs 29-50 and negative controls.
Fig. 2: SHapley Additive exPlanation (SHAP) analysis performed on predicTCR. The top 10 genes were illustrated with beeswarm plot, with each dot representing a single cell. The greyscale represents the expression and x-axis represent the SHAP value, in which a positive value contributes to reactive prediction and vice versa. Thus, e.g. for TPT1, high expression is predictive of non-reactivity, and low expression is predictive of reactivity.
Fig. 3: (A) The prediction of reactivity on all BT21 T Cells projected on a UMAP plot. (B) The reactivity score for each TCR was determined from individual T cells using probability of reactivity, as assessed by the classifier. The density plot show the distribution of TCR reactivity score and dotted line indicates threshold calculated using Fisher-Jenk natural break optimization. (C) Based on (B), a further 22 TCRs from BT21 patient were tested by co-culturing with patient-derived cell lines. CD107a was measured similarly with flow cytometry.
Fig. 4: The performance of predicTCR as shown by AUC plots on (A) PDAC, (B) Colorectal Metastatic Cancer, (C) Non-Small Cell Lung Cancer and (D) Gastrointestinal Cancer. See Table 3 for comprehensible metrics. Codes are patient codes of Table 3.
Fig. 5: (A) Prediction of tumor reactive TCR using predicTCR in the prospective patient of Example 6.4. The density plot show the distribution of TCR reactivity score and the dotted line indicates the prediction threshold calculated using Fisher-Jenk natural break optimization.

Reactivity test of predicted TCRs measured by (B) CD107a and (C) TNFα. Mock and flu TCR were included as control.

The following Examples shall merely illustrate the invention. They shall not be construed, whatsoever, to limit the scope of the invention. Exemplary protocols for performing the Examples below can also be derived from the Literature indicated below, in particular Meng et al., Sci. Transl. Med. 15, eadh9562 (2023), Lowery et al., Science 375, 877-884 (2022), Caushi. et al., Nature 596, 126-132 (2021), and Zheng et al. Cancer Cell 40, 410- 423.e7 (2022).

### Example 1: Single Cell Library Preparation

Single cell tumor suspension was FACS-sorted for CD45⁺CD3⁺ population to enrich for T cells. Single Cell library of sorted T cells was prepared using 10X Single Cell Immune Profiling Kit according to manufacturer's protocols. The resulting scVDJ and scRNA library were then sequenced on Novaseq-6k.

### Example 2: Single Cell RNA Analysis

Raw data was processed with cellranger pipeline (v6.1.2) to generate gene expression matrices. Matrices were imported into Rand analyzed using Seurat. For quality control, SoupX was used to remove background noise and miQC to removed cells with high mitochondrial gene expression. Additionally, outliers were also removed based on UMI and the number of genes. The data was then normalized using sctransform method.

### Example 3: Co-culture Assay

RNA for each individual TCR was prepared using Cellscript kit according to manufacturer's protocol. The concentration and integrity of RNA was assessed by Nanodrop and Tapestation respectively. The TCRs were then expressed by electroporation of RNA into expanded PBMC using the Lonza 4D nucleofactor device. The cells were then plated into 48-well plates containing TexMACS media supplemented with 2% AB serum and allowed to rest overnight. The electroporated cells were then co-cultured with target cells (patient-derived tumor cell line) with CD107a FACS antibody added for 5 hours, with the addition of Golgistop and Golgiplug after 1 hour. After co-culture, the cells were stained for DCM (dead cell marker), CD3, CD4, CD8a, mTCRβ and IFNγ,and then measured using FACSlyrics (BD Biosciences). Further analysis of data was done using FlowJo.

### Example 4: predicTCR Classifier Training

The normalized transcriptomic data of tested BT21 TCR (1461 cells) was used as training data input, with additional data from healthy donor PBMC (112960 cells). Healthy PBMC datasets were obtained from multiple sources to increase variability: a single donor from 10X Genomics (www.10xgenomics.com/resources/datasets/human- T cells-from-a-healthy-donor-1-k-cells-multi-v-2-2-standard-5-0-0), two donors from Szabo et al., and seven donors from Gao et al. TCR reactivity (label) based on CD107a expression in co-culture assay was converted into binary value and all healthy donor PBMCs were assumed to be non-reactive. Hyperparameter tuning was performed using Bayesian optimization (scikit-optimize) with 10 stratified k-fold cross validation for XGBoost hyperparameters 'colsample_bytree', 'gamma', `learning_rate', 'max_delta_step', 'max_depth', 'min_child_weight', 'n_estimators', 'alpha', 'lambda', 'scale_pos_weight' and 'subsample'. To prevent overfitting, a simpler model was trained by identification key genes contributing to the model using Shapley Additive exPlanations (SHAP). The final classifier (termed predicTCR) was then trained based on the top 100 SHAP features (genes) and hyperparameters were optimized as before.

### Example 5: Prediction of TCR using predicTCR

All raw data for validation of predicTCR was processed as described above. The probability of reactivity, as predicted by predicTCR, was average for each TCR clonotype (Reactive Score, which may also be referred to as "Reactivity Score"). Using Fisher-Jenks natural break optimization, the threshold for distinguishing reactive and non-reactive TCR clonotypes was determined.

### Example 6: Results

### 6.1 Proof of Principle for M L-based approach

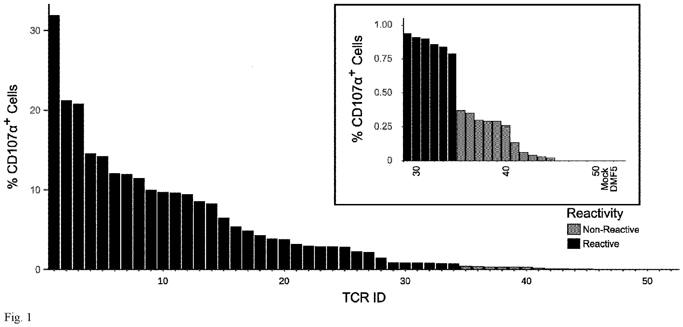

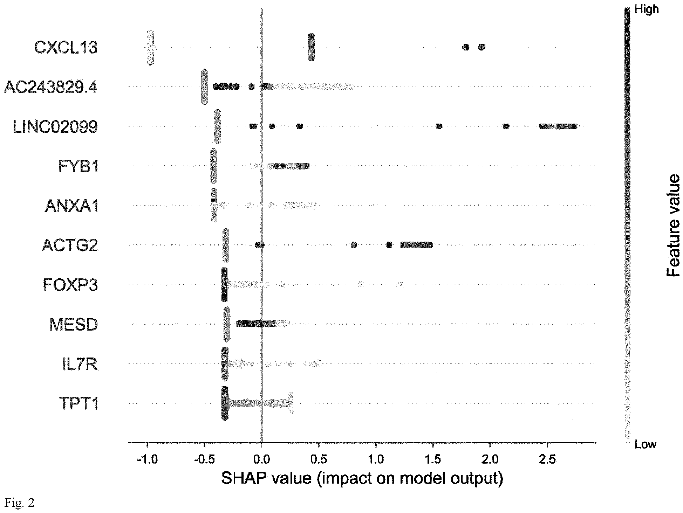

To train a machine learning(ML) based model for prediction of tumor reactive TCR, we have cloned and tested the top 50 TCRs from a melanoma brain metastasis patient (BT21) in unbiased fashion. The reactivity of TCR was then determined using co-culture assay (Figure 1). The transcriptome data was then used for training with the reactivity as label. Using various machine learning models, ML-based approach perform well (AUC > 0.85); AUC values determined were for the respective models (model: AUC): Adaboost: 0.90; Decision Tree: 0.89; Catboost: 0.90; Gaussian Naive Bayes: 0.90; K-nearest Neighbor: 0.85; Logistic Regression: 0.85; SVM (RBF): 0.86; Random Forest: 0.88; SVM (Linear): 0.86; and XGBoost: 0.92; thus, XGBoost performed best (AUC = 0.92). To train a simpler model, SHAP analysis was performed and only the top 100 genes identified via SHAP was included in retraining. The top 10 genes were identified by SHapley Additive exPlanation (SHAP) analysis(Figure 2). As shown in Figure 2, there is not in each case a direct proportionality between the expression level of a biomarker and impact on model output in the model. Without wishing to be bound by theory, it is assumed that this may be caused by non-linear contributions of biomarkers to the model (e.g. contribution only starting from and/or up to a given threshold value), and/or mutual interdependencies between biomarkers (contribution of marker A only if marker B is present at a given level).

### 6.2 Prospective Prediction of Tumor Reactive TCR

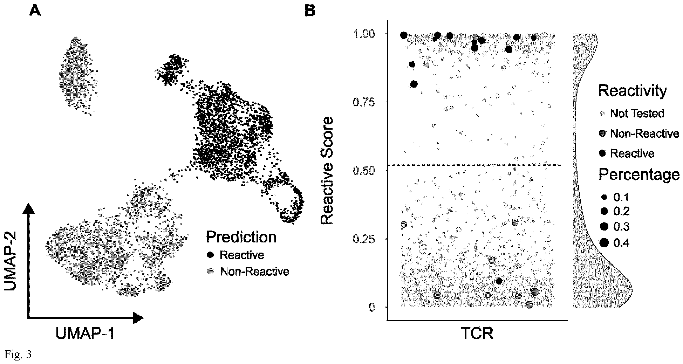

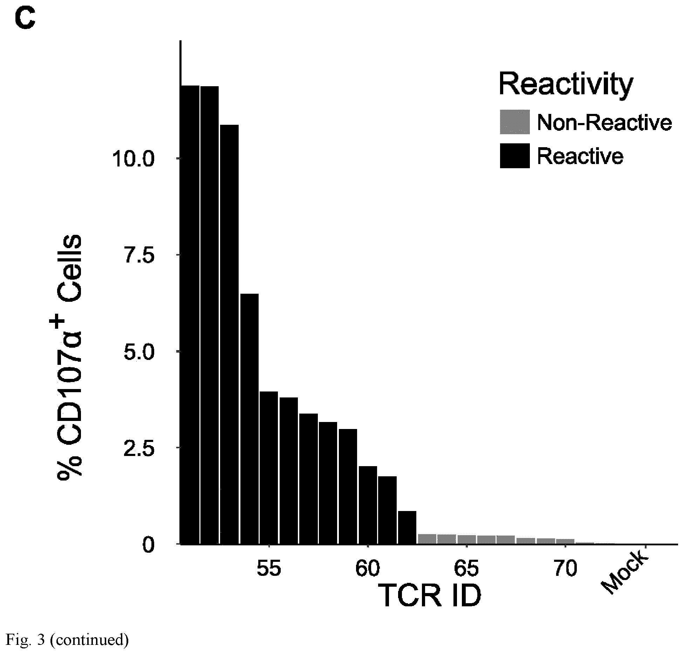

Using XGBoost, a classifier was trained using the 50 TCRs with additional PBMC healthy datasets (Szabo et al. Nat. Commun. 10:4706 (2019); Gao et al., Nat. Commun. 13:1982 (2022)) and the hyperparameters was determined using Bayesian optimization. The prediction was then performed on all T cells from BT21 (Figure 3A). A further 22 TCRs were selected based on the prediction (Figure 3B) and tested with co-culture assay (Figure 3C). 20 out of 22 (91% accuracy) of the TCR were predicted correctly (Figure 3 B,C), which is higher than all other signature-based approaches (Table 2).

### 6.3 Prediction of Tumor Reactive TCR from Diverse Tumor Type

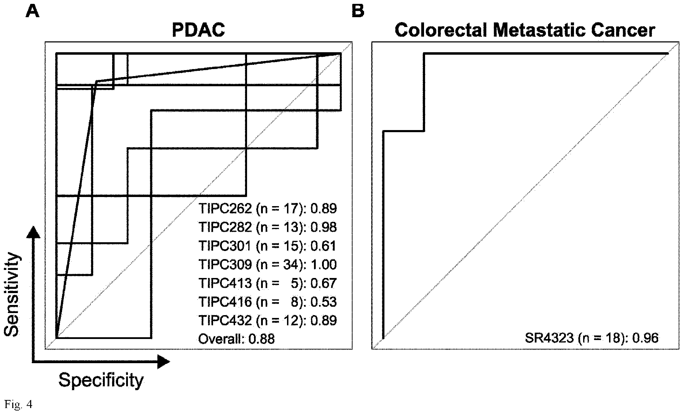

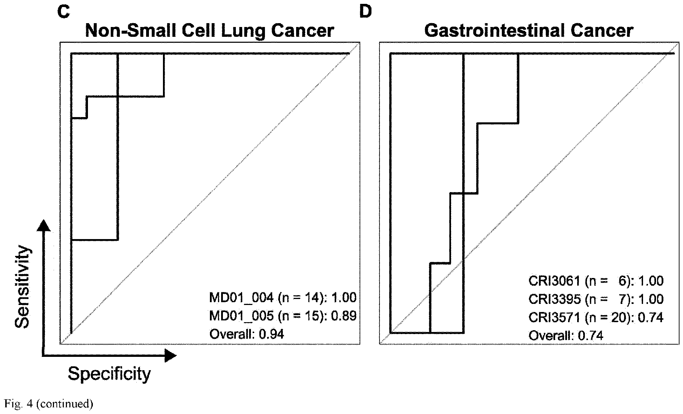

To determine the generalizability of TCR, the performance of predicTCR was evaluated using external datasets from various diverse tumor types (Table 3). When applied to pancreatic ductal adenocarcinoma, predicTCR was able to predict tumor reactive TCR well with Overall AUC of 0.88 (Figure 4A). Similarly, when applied to colorectal metastatic cancer and non-small cell lung cancer, predicTCR was also able to predict tumor-reactive TCR with AUC of 0.96 and 0.94 respectively (Figure 4B,C). Even when extending predicTCR to single cell data generated using an other technology such as smart-seq, predicTCR was also able to predict tumor-reactive TCR from gastrointestinal cancer, with AUC of 0.74. Furthermore, predicTCR performed better than all other signature based approaches (see Tables 4-7 for detailed comparisons).

### 6.4 Prospective Prediction of Tumor Reactive TCR in New Patient

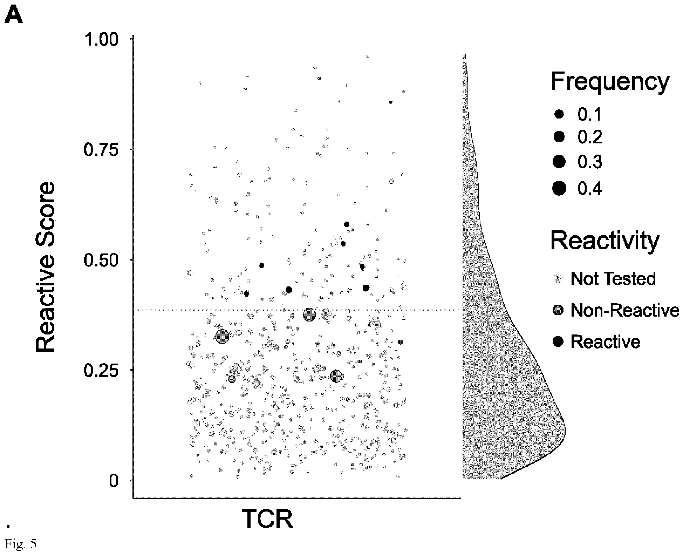

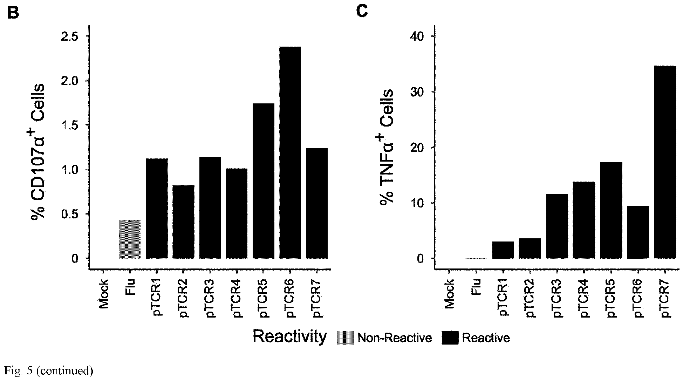

In a patient (TIPC418) of *Meng* et al., all of the 8 predicted reactive TCRs tested negative. However, predicTCR was able to identify many potential reactive TCRs (Figure 5A). Hence, based on predicTCR, we further cloned 7 TCRs from this patient and tested them with the patient-derived tumor cell line with co-culture assay. Based on CD107a and TNFα expression in flow cytometry, all 7 of the predicted TCRs were indeed reactive, further showcasing the predictability of predicTCR (Figure 5B,C).

Literature
- Bernardeau et al., (2011), J Immunol Methods, 371(1- 2):97- 105
- Bordner (2010), PLoS ONE 5(12): e14383
- Cano- Gamez et al., Nat Comm 11:, art. 1801 (2020) (doi.org/10.1038/s41467- 020- 15543-y)
- Caushi. et al., Nature 596, 126-132 (2021)
- Crompton et al., Immunol. Rev. 257, 264-276 (2014)
- Gao et al., Nat. Commun. 13:1982 (2022)
- Lowery et al., Science 375, 877-884 (2022)
- Lundberg & Lee, "A Unified Approach to Interpreting Model Predictions. in Advances in Neural Information Processing Systems" (eds. Guyon, I. et al.) vol. 30 (Curran Associates, Inc., 2017)
- Magen et al., Cell Rep 29(10):3019 (2019)
- Meng et al., Sci. Transl. Med. 15, eadh9562 (2023)
- Nielsen et al., (2004), Bioinformatics, 20 (9), 1388-1397
- Oh et al., Cell 181(7):1612 (2020)
- Poschke et al., Clin. Cancer Res. 26, 4289-4301 (2020)
- Rohaan et al., N. Engl. J. Med. 387, 2113-2125 (2022)
- Simoni et al., Nature 557, 575-579 (2018)
- Szabo et al. Nat. Commun. 10:4706 (2019)
- WO2018/209324
- WO2019/070755
- WO 2021/188954 A1
- WO 2022/200456 A1
- WO 2022/200457 A1
- Zheng et al. Cancer Cell 40, 410- 423.e7 (2022)

**Table 1: Preferred biomarkers, their full gene names, and database accession numbers. For AC243829.4, also the designation CCL3-AS1 has been used.**

| No. | Gene Name | Full Gene Name | Ensembl ID | Entrez ID | Refseq ID |
|---|---|---|---|---|---|
| 1 | CXCL13 | C-X-C motif chemokine ligand 13 | ENSG00000156234 | 10563 | NM_006419.3; NM_001371558.1 |
| 2 | AREG | amphiregulin | ENSG00000109321 | 374 | NM_001657.4 |
| 3 | MAGEH1 | MAGE family member H1 | ENSG00000187601 | 28986 | NM_014061.5 |
| 4 | FOXP3 | forkhead box P3 | ENSG00000049768 | 50943 | NM_014009.4; NM_001114377.2 |
| 5 | ANXA1 | annexin A1 | ENSG00000135046 | 301 | NM_000700.3 |
| 6 | HMGB2 | high mobility group box 2 | ENSG00000164104 | 3148 | NM_002129.4; NM_001130688.1; NM_001130689.1 |
| 7 | MIR4435-2HG | MIR4435-2 host gene | ENSG00000172965 | 541471 | NR_015395.2; NR_024373.2; NR_136161.1; NR_136162.1; NR_136163.1; NR_136164.1; NR_136165.1; NR_136166.1 |
| 8 | TMSB10 | thymosin beta 10 | ENSG00000034510 | 9168 | NM_021103.4 |
| 9 | MGAT4A | alpha-1,3-mannosyl-glycoprotein 4-beta-N-acetylglucosaminyltransferase A | ENSG00000071073 | 11320 | NM_012214.3; NM_001160154.2 |
| 10 | C12orf57 | chromosome 12 open reading frame 57 | ENSG00000111678 | 113246 | NM_138425.4; NM_001301834.1; NM_001301836.2; NM_001301837.2; NM_001301838.2 |
| 11 | GIMAP7 | GTPase; IMAP family member 7 | ENSG00000179144 | 168537 | NM_1 53236.4 |
| 12 | HSPE1 | heat shock protein family E (Hsp10) member 1 | ENSG00000115541 | 3336 | NM_002157.3 |
| 13 | IL6ST | interleukin 6 cytokine family signal transducer | ENSG00000134352 | 3572 | NM_002184.4; NM_175767.3; NM_001190981.2; NM_001364275.2; NM_001364276.2; NM_001364277.2; NM_001364278.2; NM_001364279.2 |
| 14 | STMN1 | stathmin 1 | ENSG00000117632 | 3925 | NM_203401.2; NM_203399.2; NM_005563.4; NM_001145454.3 |
| 15 | AC243829.4 (CCL3-AS1) | CCL3 Antisense RNA 1 | ENSG00000277089 | 10272485 0 | NR_186417.1 |
| 16 | IL32 | interleukin 32 | ENSG00000008517 | 9235 | NM_001012631.4; NM_004221.7; NM_001012632.3; NM_001012633.4; NM_001012634.4; NM_001012635.4; NM_001012636.2; NM_001012718.4; NM_001308078.4; NM_001369587.3; NM_001369588.3; NM_001369589.3; NM_001369590.3; NM_001369591.3; NM_001369592.3; NM_001369593.3; NM_001369595.3; NM_001369596.3; NM_001376923.1 |
| 17 | LINC02446 | long intergenic non-protein coding RNA 2446 | ENSG00000256039 | 10106003 8 | NR_146455.1; NR_146456.1 |
| 18 | IFNG | interferon gamma | ENSG00000111537 | 3458 | NM_000619.3 |
| 19 | NKG7 | natural killer cell granule protein 7 | ENSG00000105374 | 4818 | NM_005601.4; NM_001363693.2 |
| 20 | CTSC | cathepsin C | ENSG00000109861 | 1075 | NM_001814.6; NM_148170.5; NM_001114173.3 |
| 21 | TPT1 | tumor protein; translationally-controlled 1 | ENSG00000133112 | 7178 | NM_001286272.2; NM_003295.4; NM_001286273.2 |
| 22 | LYAR | Ly1 antibody reactive | ENSG00000145220 | 55646 | NM_017816.3; NM_001145725.2 |
| 23 | MIF | macrophage migration inhibitory factor | ENSG00000240972 | 4282 | NM_002415.2 |
| 24 | PALM2-AKAP2 | PALM2 and AKAP2 fusion | ENSG00000157654 | 445815 | NM_053016.6; NM_001037293.3; NM_007203.5; NM_147150.3; NM_001004065.4; NM_001198656.1; NM_001136562.3 |
| 25 | ISCU | iron-sulfur cluster assembly enzyme | ENSG00000136003 | 23479 | NM_014301.4; NM_213595.4; NM_001301140.1; NM_001301141.1; NM_001320042.1 |
| 26 | CCR8 | C-C motif chemokine receptor 8 | ENSG00000179934 | 1237 | NM_005201.4 |
| 27 | UGP2 | UDP-glucose pyrophosphorylase 2 | ENSG00000169764 | 7360 | NM_006759.4; NM_001001521.2; NM_001377524.1; NM_001377525.1; NM_001377526.1; NM_001377527.1; NM_001377528.1; NM_001377529.1 |
| 28 | EEF1B2 | eukaryotic translation elongation factor 1 beta 2 | ENSG00000114942 | 1933 | NM_001959.4; NM_021121.4; NM_001037663.2 |
| 29 | SETD7 | SET domain containing 7; histone lysine methyltransferase | ENSG00000145391 | 80854 | NM_030648.4; NM_001306199.2; NM_001306200.2 |
| 30 | LTB | lymphotoxin beta | ENSG00000227507 | 4050 | NM_002341.2; NM_009588.1 |
| 31 | CRIP1 | cysteine rich protein 1 | ENSG00000213145 | 1396 | NM_001311.5 |
| 32 | APOBEC3G | apolipoprotein B mRNA editing enzyme catalytic subunit 3G | ENSG00000239713 | 60489 | NM_021822.4; NM_001349436.1; NM_001349437.2; NM_001349438.3 |
| 33 | RBPJ | recombination signal binding protein for immunoglobulin kappa J region | ENSG00000168214 | 3516 | NM_005349.4; NM_015874.6; NM_203283.5; NM_203284.3; NM_001363577.2; NM_001374400.1; NM_001374401.1; NM_001374402.1; NM_001374403.1; NM_001379406.1; NM_001379407.1; NM_001379408.1; NM_001379409.1 |
| 34 | RGS1 | regulator of G protein signaling 1 | ENSG00000090104 | 5996 | NM_002922.4 |
| 35 | MESD | mesoderm development LRP chaperone | ENSG00000117899 | 23184 | NM_015154.3 |
| 36 | LINC02099 | long intergenic non-protein coding RNA 2099 | ENSG00000253490 | 10192945 0 | NR_125814.1; NR_125815.1; NR_125816.1 |
| 37 | IKZF3 | IKAROS family zinc finger 3 | ENSG00000161405 | 22806 | NM_012481.5; NM_183228.3; NM_183229.3; NM_183230.3; NM_183231.3; NM_183232.3; NM_001257408.2; NM_001257409.2; NM_001257410.2; NM_001257411.2; NM_001257412.2; NM_001257413.2; NM_001257414.2; NM_001284514.2; NM_001284515.2; NM_001284516.1 |
| 38 | IFT52 | intraflagellar transport 52 | ENSG00000101052 | 51098 | NM_016004.5; NM_001303458.3; NM_001303459.3; NM_001323578.2; NM_001323579.2; NM_001323580.2; NM_001323581.2 |
| 39 | AKIRIN2 | akirin 2 | ENSG00000135334 | 55122 | NM_018064.4 |
| 40 | RIOK3 | RIO kinase 3 | ENSG00000101782 | 8780 | NM_003831.5; NM_001348193.2 |
| 41 | CLIC3 | chloride intracellular channel 3 | ENSG00000169583 | 9022 | NM_004669.3 |
| 42 | ANKRD12 | ankyrin repeat domain 12 | ENSG00000101745 | 23253 | NM_015208.5; NM_001083625.3; NM_001204056.1 |
| 43 | CALR | calreticulin | ENSG00000179218 | 811 | NM_004343.4 |
| 44 | DDX5 | DEAD-box helicase 5 | ENSG00000108654 | 1655 | NM_001320595.2; NM_004396.5; NM_001320596.3; NM_001320597.2 |
| 45 | CNST | consortin; connexin sorting protein | ENSG00000162852 | 163882 | NM_152609.3; NM_001139459.2 |
| 46 | GNPDA1 | glucosamine-6-phosphate deaminase 1 | ENSG00000113552 | 10007 | NM_005471.5 |
| 47 | PKM | pyruvate kinase M1/2 | ENSG00000067225 | 5315 | NM_002654.6; NM_182470.4; NM_182471.4; NM_001206796.3; NM_001206797.3; NM_001206798.3; NM_001206799.2; NM_001316318.2; NM_001411081.1 |
| 48 | SPOCK2 | SPARC (osteonectin); cwcv and kazal like domains proteoglycan 2 | ENSG00000107742 | 9806 | NM_001134434.1; NM_014767.2; NM_001244950.2 |
| 49 | RSRP1 | arginine and serine rich protein 1 | ENSG00000117616 | 57035 | NM_020317.5; NM_001321772.2 |
| 50 | IL7R | interleukin 7 receptor | ENSG00000168685 | 3575 | NM_002185.5; NM_001410734.1 |
| 51 | KIR2DL4 | killer cell immunoglobulin like receptor; two Ig domains and long cytoplasmic tail 4 | ENSG00000189013 | 3805 | NM_002255.6; NM_001080772.2; NM_001080770.2 |
| 52 | MALAT1 | metastasis associated lung adenocarcinoma transcript 1 | ENSG00000251562 | 378938 | NR_002819.4; NR_144567.1; NR_144568.1 |
| 53 | CTLA4 | cytotoxic T-lymphocyte associated protein 4 | ENSG00000163599 | 1493 | NM_005214.5; NM_001037631.3 |
| 54 | TOX | thymocyte selection associated high mobility group box | ENSG00000198846 | 9760 | NM_014729.3 |
| 55 | RGL4 | ral guanine nucleotide dissociation stimulator like 4 | ENSG00000159496 | 266747 | NM_001329424.3; NM_153615.2; NM_001329425.2 |
| 56 | KLF6 | KLF transcription factor 6 | ENSG00000067082 | 1316 | NM_001300.6; NM_001160124.2; NM_001160125.2 |
| 57 | B2M | beta-2-microglobulin | ENSG00000166710 | 567 | NM_004048.4 |
| 58 | IFITM2 | interferon induced transmembrane protein 2 | ENSG00000185201 | 10581 | NM_006435.3 |
| 59 | ABI3 | ABI family member 3 | ENSG00000108798 | 51225 | NM_001135186.2; NM_016428.3 |
| 60 | CST7 | cystatin F | ENSG00000077984 | 8530 | NM_003650.4 |
| 61 | PTPN11 | protein tyrosine phosphatase non-receptor type 11 | ENSG00000179295 | 5781 | NM_002834.5; NM_080601.3; NM_001330437.2; NM_001374625.1 |
| 62 | BTG1 | BTG anti-proliferation factor 1 | ENSG00000133639 | 694 | NM_001731.3 |
| 63 | CCNH | cyclin H | ENSG00000134480 | 902 | NM_001239.4; NM_001199189.2; NM_001363539.2; NM_001364075.2; NM_001364076.2 |
| 64 | ITSN2 | intersectin 2 | ENSG00000198399 | 50618 | NM_006277.3; NM_147152.3; NM_019595.4; NM_001348181.2; NM_001348182.2; NM_001348183.2; NM_001348184.2; NM_001348185.2; NM_001348186.2 |
| 65 | RAD21 | RAD21 cohesin complex component | ENSG00000164754 | 5885 | NM_006265.3 |
| 66 | CCL3 | C-C motif chemokine ligand 3 | ENSG00000277632 | 6348 | NM_002983.3 |
| 67 | HMOX1 | heme oxygenase 1 | ENSG00000100292 | 3162 | NM_002133.3 |
| 68 | FYB1 | FYN binding protein 1 | ENSG00000082074 | 2533 | NM_001465.6; NM_199335.5; NM_001243093.2; NM_001349333.2; NM_018594.2 |
| 69 | DDX21 | DExD-box helicase 21 | ENSG00000165732 | 9188 | NM_004728.4; NM_001256910.2; NM_001410932.1 |
| 70 | GALNT2 | polypeptide N-acetylgalactosaminyltransferase 2 | ENSG00000143641 | 2590 | NM_004481.5; NM_001291866.2 |
| 71 | HSPD1 | heat shock protein family D (Hsp60) member 1 | ENSG00000144381 | 3329 | NM_002156.5; NM_199440.2 |
| 72 | GZMA | granzyme A | ENSG00000145649 | 3001 | NM_006144.4 |
| 73 | JAK1 | Janus kinase 1 | ENSG00000162434 | 3716 | NM_002227.4; NM_001320923.2; NM_001321852.2; NM_001321853.2; NM_001321854.2; NM_001321855.2; NM_001321856.2; NM_001321857.2 |
| 74 | CHST12 | carbohydrate sulfotransferase 12 | ENSG00000136213 | 55501 | NM_001243794.2; NM_001243795.2; NM_018641.5 |
| 75 | ADAM 19 | ADAM metallopeptidase domain 19 | ENSG00000135074 | 8728 | NM_033274.5 |
| 76 | PRF1 | perforin 1 | ENSG00000180644 | 5551 | NM_005041.6; NM_001083116.3 |
| 77 | CD63 | CD63 molecule | ENSG00000135404 | 967 | NM_001780.6; NM_001257389.2; NM_001257390.2; NM_001257391.2; NM_001257392.1; NM_001257400.2; NM_001257401.2; NM_001267698.2; NM_001413281.1; NM_001413282.1; NM_001413283.1; NM_001413284.1; NM_001413285.1; NM_001413286.1; NM_001413287.1; NM_001413288.1 |
| 78 | TM2D3 | TM2 domain containing 3 | ENSG00000184277 | 80213 | NM_078474.3; NM_025141.4; NM_001307960.2; NM_001308026.2 |
| 79 | CASP8 | caspase 8 | ENSG00000064012 | 841 | NM_001228.5; NM_033355.4; NM_033356.4; NM_001080124.2; |
| | | | | | NM_001080125.2; NM_001372051.1; NM_001400642.1; NM_001400645.1; NM_001400648.1; NM_001400651.1; NM_001400653.1; NM_001400654.1 ; NM_001400655.1; NM_001400656.1; NM_001400657.1; NM_001400658.1; NM_001400659.1; NM_001400660.1; NM_001400661.1; NM_001400662.1; NM_001400663.1; NM_001400664.1; NM_001400665.1; NM_001400666.1; NM_001400667.1; NM_001400668.1; NM_001400669.1; NM_001400670.1 ; NM_001400671.1; NM_001400672.1; NM_001400673.1; NM_001400674.1; NM_001400675.1; NM_001400676.1; NM_001400677.1; NM_001400678.1; NM_001400679.1; NM_001400680.1; NM_001400750.1; NM_001400751.1 |
| 80 | CARS1 | Cysteinyl-TRNA Synthetase 1 | ENSG00000110619 | 833 | NM_139273.4; NM_001751.6; NM_001014437.3; NM_001194997.2; NM_001378136.1; NM_001378137.1; NM_001378138.1; NM_001378139.1; NM_001378140.1 |
| 81 | ACP5 | acid phosphatase 5; tartrate resistant | ENSG00000102575 | 54 | NM_001111035.3; NM_001111034.3; NM_001111036.3; NM_001611.5; NM_001322023.2 |
| 82 | PTPN7 | protein tyrosine phosphatase non-receptor type 7 | ENSG00000143851 | 5778 | NM_002832.4; NM_080588.3; NM_001199797.2; NM_001364877.2; NM_001364878.1 |
| 83 | RHOH | ras homolog family member H | ENSG00000168421 | 399 | NM_001278359.2; NM_001278360.2; NM_001278361.2; NM_001278362.2; NM_001278363.2; NM_004310.5; NM_001278364.2; NM_001278365.2; NM_001278366.2; NM_001278367.2; NM_001278368.2; NM_001278369.2 |
| 84 | HAPLN3 | hyaluronan and proteoglycan link protein 3 | ENSG00000140511 | 145864 | NM_001307952.2; NM_178232.4 |
| 85 | CSTB | cystatin B | ENSG00000160213 | 1476 | NM_000100.4 |
| 86 | TLE5 | TLE family member 5; transcriptional modulator | ENSG00000104964 | 166 | NM_198969.1; NM_001130.6; NM_198970.2 |
| 87 | ACTG2 | actin gamma 2; smooth muscle | ENSG00000163017 | 72 | NM_001615.4; NM_001199893.2 |
| 88 | CD7 | CD7 molecule | ENSG00000173762 | 924 | NM_006137.7 |
| 89 | IL2RA | interleukin 2 receptor subunit alpha | ENSG00000134460 | 3559 | NM_000417.3; NM_001308242.2; NM_001308243.2 |
| 90 | CD99 | CD99 molecule (Xg blood group) | ENSG00000002586 | 4267 | NM_002414.5; NM_001122898.3; NM_001321367.2; NM_001321368.2; NM_001321369.2; NM_001321370.2 |
| 91 | CARHSP1 | calcium regulated heat stable protein 1 | ENSG00000153048 | 23589 | NM_014316.4; NM_001042476.2; NM_001278260.2; NM_001278261.2; NM_001278262.2; NM_001278263.1; NM_001278264.2; NM_001278265.2; NM_001278266.2 |
| 92 | TMSB4X | thymosin beta 4 X-linked | ENSG00000205542 | 7114 | NM_021109.4 |
| 93 | PSMB9 | proteasome 20S subunit beta 9 | ENSG00000240065 | 5698 | NM_002800.5 |
| 94 | CCR4 | C-C motif chemokine receptor 4 | ENSG00000183813 | 1233 | NM_005508.5 |
| 95 | AKAP13 | A-kinase anchoring protein 13 | ENSG00000170776 | 11214 | NM_006738.6; NM_007200.5; NM_001270546.1 |
| 96 | RABAC1 | Rab acceptor 1 | ENSG00000105404 | 10567 | NM_006423.3 |
| 97 | HLA-DRA | major histocompatibility complex; class II; DR alpha | ENSG00000204287 | 3122 | NM_019111.5 |
| 98 | FTH1 | ferritin heavy chain 1 | ENSG00000167996 | 2495 | NM_002032.3 |
| 99 | CD27 | CD27 molecule | ENSG00000139193 | 939 | NM_001413263.1; NM_001242.5; NM_001413264.1; NM_001413265.1; NM_001413266.1; NM_001413267.1; NM_001413268.1 |
| 100 | PCM1 | pericentriolar material 1 | ENSG00000078674 | 5108 | NM_006197.4; NM_001315507.2; NM_001315508.2; NM_001352632.2; NM_001352633.2; NM_001352634.2; NM_001352635.2; NM_001352636.2; NM_001352637.2; NM_001352638.2; NM_001352639.2; NM_001352640.2; NM_001352641.2; NM_001352642.2; NM_001352643.2; NM_001352644.2; NM_001352645.2; NM_001352646.2; NM_001352647.2; NM_001352648.2; NM_001352649.2; NM_001352650.2; NM_001352651.2; NM_001352652.2; NM_001352653.2; NM_001352654.2; NM_001352655.2; NM_001352656.2; NM_001352657.2; NM_001352658.2; NM_001352659.2; NM_001352660.2 |

**Table 2: Performance of predicTCR against gene signature approach on predicting BT21 tumour reactive TCR**

| **Methods** | **Threshold** | **TP** | **FP** | **TN** | **FN** | **Accuracy** | **G-Mean** | **AUC** |
|---|---|---|---|---|---|---|---|---|
| **predicTCR** | 0.52 | 12 | 1 | 8 | 1 | 0.91 | 0.91 | 0.92 |
| **NeoTCR8** | 0.39 | 2 | 0 | 9 | 11 | 0.5 | 0.15 | 0.87 |
| **Hanada** | 0.43 | 13 | 5 | 4 | 0 | 0.82 | 0.67 | 0.72 |
| **Caushi** | 0.46 | 13 | 5 | 4 | 0 | 0.77 | 0.67 | 0.72 |
| **Meng TR30** | 0.37 | 12 | 4 | 5 | 1 | 0.77 | 0.72 | 0.85 |

**Table 3: Performance of predicTCR in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.39 | 5 | 0 | 0 | 1 | 0.83 | NA | NA |
| TIPC262 | Meng et al. | PDAC | Cell Line | 10X | 0.39 | 9 | 2 | 6 | 0 | 0.88 | 0.87 | 0.89 |
| TIPC282 | Meng et al. | PDAC | Cell Line | 10X | 0.39 | 8 | 1 | 4 | 0 | 0.92 | 0.89 | 0.98 |
| TIPC301 | Meng et al. | PDAC | Cell Line | 10X | 0.39 | 0 | 1 | 11 | 3 | 0.73 | 0.00 | 0.61 |
| TIPC309 | Meng et al. | PDAC | Cell Line | 10X | 0.39 | 21 | 0 | 13 | 0 | 1.00 | 1.00 | 1.00 |
| TIPC413 | Meng et al. | PDAC | PDX | 10X | 0.39 | 1 | 0 | 3 | 1 | 0.80 | 0.71 | 0.67 |
| TIPC416 | Meng et al. | PDAC | Cell Line | 10X | 0.39 | 3 | 1 | 2 | 2 | 0.63 | 0.63 | 0.53 |
| TIPC418 | Meng et al. | PDAC | Cell Line | 10X | 0.39 | 0 | 1 | 7 | 0 | 0.88 | NA | NA |
| TIPC432 | Meng et al. | PDAC | Cell Line | 10X | 0.39 | 8 | 0 | 3 | 1 | 0.92 | 0.94 | 0.89 |
| TIPC Overall | Meng et al. | PDAC | Cell Line | 10X | NA | 55 | 6 | 49 | 8 | 0.88 | 0.88 | 0.88 |
| SR4323 | Lowery et al. | Colon-Met | TMG | 10X | 0.45 | 11 | 3 | 4 | 0 | 0.83 | 0.76 | 0.96 |
| MD01-004 | Caushi et al. | NSCLC | Peptide | 10X | 0.57 | 8 | 2 | 4 | 0 | 0.86 | 0.82 | 1.00 |
| MD01-005 | Caushi et al. | NSCLC | Peptide | 10X | 0.45 | 1 | 0 | 12 | 2 | 0.87 | 0.58 | 0.89 |
| MD043-011 | Caushi et al. | NSCLC | Peptide | 10X | 0.54 | 2 | 0 | 0 | 0 | 1.00 | NA | NA |
| MD Overall | Caushi et al. | NSCLC | Peptide | 10X | NA | 11 | 2 | 16 | 2 | 0.87 | 0.87 | 0.94 |
| CRI3061 | Zheng et al. | GI (PDAC) | TMG + Pep | SS2* | 0.16 | 2 | 0 | 4 | 0 | 1.00 | 1.00 | 1.00 |
| CRI3244 | Zheng et al. | GI (PDAC) | TMG + Pep | SS2* | 0.24 | 0 | 1 | 7 | 0 | 0.88 | NA | NA |
| CRI3281 | Zheng et al. | GI (Bile Duct) | TMG + Pep | SS2 | 0.15 | 0 | 2 | 2 | 0 | 0.50 | NA | NA |
| CRI3395 | Zheng et al. | GI (Bile Duct) | TMG + Pep | SS2* | 0.15 | 1 | 1 | 5 | 0 | 0.86 | 0.91 | 1.00 |
| CRI3571 | Zheng et al. | GI (Bile Duct) | TMG + Pep | SS2* | 0.15 | 1 | 14 | 5 | 0 | 0.30 | 0.51 | 0.74 |
| CRI Overall | Zheng et al. | GI | TMG + Pep | SS2 | NA | 4 | 18 | 23 | 0 | 0.60 | 0.78 | 0.74 |

**Table 4: Performance of NeoTCR8 in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.44 | 0 | 0 | 0 | 6 | 0.00 | NA | NA |
| TIPC262 | Meng et al. | PDAC | Cell Line | 10X | 0.44 | 1 | 0 | 8 | 8 | 0.53 | 0.33 | 0.60 |
| TIPC282 | Meng et al. | PDAC | Cell Line | 10X | 0.44 | 0 | 0 | 5 | 8 | 0.38 | 0.00 | 0.45 |
| TIPC301 | Meng et al. | PDAC | Cell Line | 10X | 0.44 | 0 | 0 | 12 | 3 | 0.80 | 0.00 | 0.42 |
| TIPC309 | Meng et al. | PDAC | Cell Line | 10X | 0.44 | 0 | 0 | 13 | 21 | 0.38 | 0.00 | 0.95 |
| TIPC413 | Meng et al. | PDAC | PDX | 10X | 0.44 | 0 | 0 | 3 | 2 | 0.60 | 0.00 | 0.33 |
| TIPC416 | Meng et al. | PDAC | Cell Line | 10X | 0.44 | 0 | 1 | 2 | 5 | 0.25 | 0.00 | 0.73 |
| TIPC418 | Meng et al. | PDAC | Cell Line | 10X | 0.44 | 0 | 1 | 7 | 0 | 0.88 | NA | NA |
| TIPC432 | Meng et al. | PDAC | Cell Line | 10X | 0.44 | 1 | 0 | 3 | 8 | 0.33 | 0.33 | 0.56 |
| TIPC Overall | Meng et al. | PDAC | Cell Line | 10X | NA | 2 | 2 | 53 | 61 | 0.47 | 0.03 | 0.65 |
| SR4323 | Lowery et al. | Colon-Met | TMG | 10X | 0.33 | 11 | 0 | 7 | 0 | 1.00 | 1.00 | 1.00 |
| MD01-004 | Caushi et al. | NSCLC | Peptide | 10X | NA* | 0 | 0 | 6 | 8 | 0.43 | 0.00 | 0.50 |
| MD01-005 | Caushi et al. | NSCLC | Peptide | 10X | 0.33 | 0 | 0 | 12 | 3 | 0.80 | 0.00 | 0.50 |
| MD043-011 | Caushi et al. | NSCLC | Peptide | 10X | NA* | 0 | 0 | 0 | 2 | 0.00 | NA | NA |
| MD Overall | Caushi et al. | NSCLC | Peptide | 10X | NA | 0 | 0 | 18 | 13 | 0.58 | 0.00 | 0.50 |
| CRI3061 | Zheng et al. | GI (PDAC) | TMG + Pep | SS2* | NA* | 0 | 0 | 4 | 2 | 0.67 | 0.00 | 0.50 |
| CRI3244 | Zheng et al. | GI (PDAC) | TMG + Pep | SS2* | NA* | 0 | 0 | 8 | 0 | 1.00 | NA | NA |
| CRI3281 | Zheng et al. | GI (Bile Duct) | TMG + Pep | SS2 | NA* | 0 | 0 | 4 | 0 | 1.00 | NA | NA |
| CRI3395 | Zheng et al. | GI (Bile Duct) | TMG + Pep | SS2* | NA* | 0 | 0 | 6 | 1 | 0.86 | 0.00 | 0.50 |
| CRI3571 | Zheng et al. | GI (Bile Duct) | TMG + Pep | SS2* | NA* | 0 | 0 | 19 | 1 | 0.95 | 0.00 | 0.50 |
| CRI Overall | Zheng et al. | GI | TMG + Pep | SS2 | NA | 0 | 0 | 41 | 4 | 0.91 | 0.00 | 0.50 |

**Table 5: Performance of Hanada et al. gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.45 | 5 | 0 | 0 | 1 | 0.83 | NA | NA |
| TIPC262 | Meng et al. | PDAC | Cell Line | 10X | 0.45 | 9 | 5 | 3 | 0 | 0.71 | 0.61 | 0.69 |
| TIPC282 | Meng et al. | PDAC | Cell Line | 10X | 0.45 | 8 | 3 | 2 | 0 | 0.77 | 0.63 | 0.70 |
| TIPC301 | Meng et al. | PDAC | Cell Line | 10X | 0.45 | 0 | 4 | 8 | 3 | 0.53 | 0.00 | 0.33 |
| TIPC309 | Meng et al. | PDAC | Cell Line | 10X | 0.45 | 21 | 0 | 13 | 0 | 1.00 | 1.00 | 1.00 |
| TIPC413 | Meng et al. | PDAC | PDX | 10X | 0.45 | 2 | 1 | 2 | 0 | 0.80 | 0.82 | 0.83 |
| TIPC416 | Meng et al. | PDAC | Cell Line | 10X | 0.45 | 2 | 1 | 2 | 3 | 0.50 | 0.52 | 0.53 |
| TIPC418 | Meng et al. | PDAC | Cell Line | 10X | 0.45 | 0 | 5 | 3 | 0 | 0.38 | NA | NA |
| TIPC432 | Meng et al. | PDAC | Cell Line | 10X | 0.45 | 8 | 0 | 3 | 1 | 0.92 | 0.94 | 0.94 |
| TIPC Overall | Meng et al. | PDAC | Cell Line | 10X | NA | 55 | 19 | 36 | 8 | 0.77 | 0.76 | 0.76 |
| SR4323 | Lowery et al. | Colon-Met | TMG | 10X | 0.33 | 11 | 5 | 2 | 0 | 0.72 | 0.53 | 0.64 |
| MD01-004 | Caushi et al. | NSCLC | Peptide | 10X | 0.33 | 5 | 0 | 6 | 3 | 0.79 | 0.79 | 0.81 |
| MD01-005 | Caushi et al. | NSCLC | Peptide | 10X | 0.42 | 1 | 1 | 11 | 2 | 0.80 | 0.55 | 0.93 |
| MD043-011 | Caushi et al. | NSCLC | Peptide | 10X | 0.45 | 2 | 0 | 0 | 0 | 1.00 | NA | NA |
| MD Overall | Caushi et al. | NSCLC | Peptide | 10X | NA | 8 | 1 | 17 | 5 | 0.81 | 0.76 | 0.86 |
| CRI3061 | Zheng et al. | GI (PDAC) | TMG + Pep | SS2* | 0.43 | 2 | 0 | 4 | 0 | 1.00 | 1.00 | 1.00 |
| CRI3244 | Zheng et al. | GI (PDAC) | TMG + Pep | SS2* | 0.56 | 0 | 7 | 1 | 0 | 0.13 | NA | NA |
| CRI3281 | Zheng et al. | GI (Bile Duct) | TMG + Pep | SS2 | 0.50 | 0 | 4 | 0 | 0 | 0.00 | NA | NA |
| CRI3395 | Zheng et al. | GI (Bile Duct) | TMG + Pep | SS2* | NA** | 1 | 6 | 0 | 0 | 0.14 | 0.00 | 0.67 |
| CRI3571 | Zheng et al. | GI (Bile Duct) | TMG + Pep | SS2* | 0.50 | 1 | 17 | 2 | 0 | 0.15 | 0.32 | 0.84 |
| CRI Overall | Zheng et al. | GI | TMG + Pep | SS2 | NA | 4 | 34 | 7 | 0 | 0.24 | 0.41 | 0.54 |

**Table 6: Performance of Caushi et al. gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.50 | 6 | 0 | 0 | 0 | 1.00 | NA | NA |
| TIPC262 | Meng et al. | PDAC | Cell Line | 10X | 0.50 | 9 | 8 | 0 | 0 | 0.53 | 0.00 | 0.50 |
| TIPC282 | Meng et al. | PDAC | Cell Line | 10X | 0.50 | 8 | 5 | 0 | 0 | 0.62 | 0.00 | 0.50 |
| TIPC301 | Meng et al. | PDAC | Cell Line | 10X | 0.50 | 3 | 12 | 0 | 0 | 0.20 | 0.00 | 0.50 |
| TIPC309 | Meng et al. | PDAC | Cell Line | 10X | 0.50 | 21 | 13 | 0 | 0 | 0.00 | 0.00 | 0.50 |
| TIPC413 | Meng et al. | PDAC | PDX | 10X | 0.50 | 2 | 2 | 1 | 0 | 0.60 | 0.58 | 0.67 |
| TIPC416 | Meng et al. | PDAC | Cell Line | 10X | 0.50 | 5 | 3 | 0 | 0 | 0.63 | 0.00 | 0.50 |
| TIPC418 | Meng et al. | PDAC | Cell Line | 10X | 0.50 | 0 | 8 | 0 | 0 | 0.00 | NA | NA |
| TIPC432 | Meng et al. | PDAC | Cell Line | 10X | 0.50 | 9 | 3 | 0 | 0 | 0.75 | 0.00 | 0.50 |
| TIPC Overall | Meng et al. | PDAC | Cell Line | 10X | NA | 63 | 54 | 1 | 0 | 0.54 | 0.13 | 0.51 |
| SR4323 | Lowery et al. | Colon-Met | TMG | 10X | 0.50 | 11 | 7 | 0 | 0 | 0.61 | 0.00 | 0.50 |
| MD01-004 | Caushi et al. | NSCLC | Peptide | 10X | 0.41 | 8 | 5 | 1 | 0 | 0.64 | 0.41 | 0.67 |
| MD01-005 | Caushi et al. | NSCLC | Peptide | 10X | 0.53 | 0 | 0 | 12 | 3 | 0.80 | 0.00 | 0.97 |
| MD043-011 | Caushi et al. | NSCLC | Peptide | 10X | 0.50 | 2 | 0 | 0 | 0 | 1.00 | NA | NA |
| MD Overall | Caushi et al. | NSCLC | Peptide | 10X | NA | 10 | 5 | 13 | 3 | 0.74 | 0.75 | 0.83 |
| CRI3061 | Zheng et al. | GI (PDAC) | TMG + Pep | SS2* | 0.50 | 2 | 4 | 0 | 0 | 0.33 | 0.00 | 0.63 |
| CRI3244 | Zheng et al. | GI (PDAC) | TMG + Pep | SS2* | 0.50 | 0 | 8 | 0 | 0 | 0.00 | NA | NA |
| CRI3281 | Zheng et al. | GI (Bile Duct) | TMG + Pep | SS2 | NA** | 0 | 4 | 0 | 0 | 0.00 | NA | NA |
| CRI3395 | Zheng et al. | GI (Bile Duct) | TMG + Pep | SS2* | 0.58 | 1 | 6 | 0 | 0 | 0.14 | 0.00 | 0.50 |
| CRI3571 | Zheng et al. | GI (Bile Duct) | TMG + Pep | SS2* | 0.75 | 1 | 18 | 1 | 0 | 0.10 | 0.23 | 0.53 |
| CRI Overall | Zheng et al. | GI | TMG + Pep | SS2 | NA | 4 | 40 | 1 | 0 | 0.11 | 0.16 | 0.57 |

**Table 7: Performance of TR30 gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.47 | 5 | 0 | 0 | 1 | 0.83 | NA | NA |
| TIPC262 | Meng et al. | PDAC | Cell Line | 10X | 0.47 | 8 | 0 | 8 | 1 | 0.94 | 0.94 | 0.99 |
| TIPC282 | Meng et al. | PDAC | Cell Line | 10X | 0.47 | 2 | 0 | 5 | 6 | 0.54 | 0.50 | 0.93 |
| TIPC301 | Meng et al. | PDAC | Cell Line | 10X | 0.47 | 0 | 1 | 11 | 3 | 0.73 | 0.00 | 0.53 |
| TIPC309 | Meng et al. | PDAC | Cell Line | 10X | 0.47 | 21 | 0 | 13 | 0 | 1.00 | 1.00 | 1.00 |
| TIPC413 | Meng et al. | PDAC | PDX | 10X | 0.47 | 1 | 0 | 3 | 1 | 0.80 | 0.71 | 1.00 |
| TIPC416 | Meng et al. | PDAC | Cell Line | 10X | 0.47 | 1 | 1 | 2 | 4 | 0.38 | 0.37 | 0.47 |
| TIPC418 | Meng et al. | PDAC | Cell Line | 10X | 0.47 | 0 | 3 | 5 | 0 | 0.63 | NA | NA |
| TIPC432 | Meng et al. | PDAC | Cell Line | 10X | 0.47 | 7 | 0 | 3 | 2 | 0.83 | 0.88 | 0.89 |
| TIPC Overall | Meng et al. | PDAC | Cell Line | 10X | NA | 45 | 5 | 50 | 18 | 0.81 | 0.81 | 0.88 |
| SR4323 | Lowery et al. | Colon-Met | TMG | 10X | 0.22 | 11 | 5 | 2 | 0 | 0.72 | 0.53 | 0.84 |
| MD01-004 | Caushi et al. | NSCLC | Peptide | 10X | 0.27 | 8 | 3 | 3 | 0 | 0.79 | 0.71 | 0.94 |
| MD01-005 | Caushi et al. | NSCLC | Peptide | 10X | 0.23 | 3 | 4 | 8 | 0 | 0.73 | 0.82 | 1.00 |
| MD043-011 | Caushi et al. | NSCLC | Peptide | 10X | 0.33 | 2 | 0 | 0 | 0 | 1.00 | NA | NA |
| MD Overall | Caushi et al. | NSCLC | Peptide | 10X | NA | 13 | 7 | 11 | 0 | 0.77 | 0.78 | 0.98 |
| CRI3061 | Zheng et al. | GI (PDAC) | TMG + Pep | SS2* | 0.20 | 2 | 2 | 2 | 0 | 0.67 | 0.71 | 1.00 |
| CRI3244 | Zheng et al. | GI (PDAC) | TMG + Pep | SS2* | 0.28 | 0 | 7 | 1 | 0 | 0.13 | NA | NA |
| CRI3281 | Zheng et al. | GI (Bile Duct) | TMG + Pep | SS2 | 0.28 | 0 | 3 | 1 | 0 | 0.25 | NA | NA |
| CRI3395 | Zheng et al. | GI (Bile Duct) | TMG + Pep | SS2* | 0.28 | 1 | 5 | 1 | 0 | 0.29 | 0.41 | 0.83 |
| CRI3571 | Zheng et al. | GI (Bile Duct) | TMG + Pep | SS2* | 0.29 | 1 | 17 | 2 | 0 | 0.15 | 0.32 | 0.63 |
| CRI Overall | Zheng et al. | GI | TMG + Pep | SS2 | NA | 4 | 34 | 7 | 0 | 0.24 | 0.41 | 0.52 |

## Claims

1. A method for identifying at least one biomarker of a T-cell reactive to target cells of a subject (reactive T-cells), said target cells presenting at least one T-cell activating antigen, the method comprising
(a) determining expression of a plurality of genes in T cells of said subject;
(b) determining reactivities of T cell receptors (TCR) of said T cells to said target cells; and,
(c) based on the results of steps (a) and (b), identifying at least one biomarker of a reactive T cell.

2. The method of claim 1, wherein in step (a) expression of at least 50, preferably at least 250, more preferably at least 1000, genes is determined..

3. The method of claim 1 or 2, wherein step (b) comprises determining the reactivities of at least 10, preferably at least 25, more preferably at least 50, TCRs to said target cells; and/or comprises step (b2) determining said reactivities by determining CD107a+ expression and/or IFN-gamma secretion of tester T cells, and/or lysis of target cells, and/or comprises step (b3) assigning numerical reactivity values to the reactivities of the TCRs determined in step (b), preferably based on the determination in step (b2).

4. The method of any one of claims 1 to 3, wherein step (c) comprises allocating gene expression data of step (a) to reactivities of corresponding TCRs determined in step (b).

5. The method of any one of claims 1 to 4, wherein step (c) comprises step (c1) providing a trained computer-implemented trainable model for identifying a reactive T cell, preferably using a computer-implemented method according any one of claims 7 to 12.

6. The method of any one of claims 1 to 5, wherein said target cells are cancer cells, preferably wherein said cancer cells form at least one tumor and wherein more preferably said T cells are primary tumor infiltrating and/or metastasis infiltrating T cells.

7. A computer-implemented method of training at least one trainable model for identifying a T-cell reactive to target cells of a subject presenting a T-cell activating antigen (reactive T-cell), the method comprising:
(A) providing the trainable model;
(B) retrieving allocated training data, the allocated training data comprising gene expression data of a plurality of genes of T cells allocated to reactivities of the T cell receptors (TCRs) of said T cells to said target cells; and
(C) training the trainable model on the allocated training data.

8. A computer-implemented method of providing a trained model for identifying a reactive T cell, said method comprising the steps of the method of claim 7, and further step (D), thereby providing a trained model for identifying a reactive T cell.

9. The method of claim 7 or 8, wherein the trainable model is selected from a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model, preferably wherein the trainable model is a trainable XGBoost model.

10. The method of any one of claims 7 to 9, wherein the gene expression data of the allocated training data in step (B) are derived from tumor-infiltrating lymphocytes and are, preferably, obtained by single cell expression analysis, preferably by mRNA analysis.

11. The method of any one of claims 7 to 10, wherein said method comprises a step of feature selection based on Shapley additive explanation (SHAP) values and repetition of hyperparameter tuning using the selected features.

12. The method of any one of claims 1 to 11, wherein the plurality of genes is at least 20, preferably at least 30, more preferably at least 38, more preferably at least 48, most preferably is at least 100, preferably wherein said genes are selected from the group consisting of CXCL13, AREG, MAGEH1, FOXP3, ANXA1, HMGB2, MIR4435-2HG, TMSB10, MGAT4A, C12orf57, GIMAP7, HSPE1, IL6ST, STMN1, AC243829.4/CCL3-AS1, IL32, LINC02446, IFNG, NKG7, CTSC, TPT1, LYAR, MIF, PALM2-AKAP2, ISCU, CCR8, UGP2, EEF1B2, SETD7, LTB, CRIP1, APOBEC3G, RBPJ, RGS1, MESD, LINC02099, IKZF3, IFT52, AKIRIN2, RIOK3, CLIC3, ANKRD12, CALR, DDX5, CNST, GNPDA1, PKM, SPOCK2, RSRP1, IL7R, KIR2DL4, MALAT1, CTLA4, TOX, RGL4, KLF6, B2M, IFITM2, ABI3, CST7, PTPN11, BTG1, CCNH, ITSN2, RAD21, CCL3, HMOX1, FYB1, DDX21, GALNT2, HSPD1, GZMA, JAK1, CHST12, ADAM19, PRF1, CD63, TM2D3, CASP8, CARS1, ACP5, PTPN7, RHOH, HAPLN3, CSTB, TLE5, ACTG2, CD7, IL2RA, CD99, CARHSP1, TMSB4X, PSMB9, CCR4, AKAP13, RABAC1, HLA-DRA, FTH1, CD27, and PCM1.

13. A computer-implemented method of predicting reactivity of a T cell receptor to target cells of a subject, the method comprising
(I) retrieving at least one trained model, preferably obtained according to the method according to any one of claims 8 to 11;
(II) retrieving gene expression data from T cells of said subject, the gene expression data comprising at least one predetermined target set of relevant genes; and
(III) applying the trained model to the gene expression data of step (II), thereby predicting reactivities of T cell receptors (TCRs) of said T cells to said target cells.

14. A system comprising a processor, said processor being configured for performing at least steps (B) and (C) of a method according to any one of claims 7 to 11 and/or at least step (III) of the method according to claim 13.

15. A trained model obtained or obtainable by a method according to any one of claims 7 to 11 tangibly embedded on a data carrier.
